Europäisches Patentamt

European Patent Office

Office européen des brevets

(19)

(11) **EP 0 217 404 B2**

(12) **NEW EUROPEAN PATENT SPECIFICATION**

(45) Date of publication and mention
of the opposition decision:
**07.01.1999 Bulletin 1999/01**

(45) Mention of the grant of the patent:
**15.01.1992 Bulletin 1992/03**

(21) Application number: **86113671.1**

(22) Date of filing: **03.10.1986**

(51) Int Cl.$^6$: **C12P 21/02**, A61K 38/00,
C12N 15/27

(54) **Pharmaceutical composition containing a human granulocyte colony stimulating factor for the treatment of leukopenia**

Einen menschlichen Granulozytkoloniereizfaktor für die Behandlung von Leukopenien enthaltende pharmazeutische Zubereitung

Composition pharmaceutique contenant un facteur stimulant les colonies de granulocyte pour le traitement de leucopénies

(84) Designated Contracting States:
**AT BE CH DE ES FR GB IT LI NL SE**

(30) Priority: **04.10.1985 JP 220450/85**
**02.06.1986 JP 125660/86**

(43) Date of publication of application:
**08.04.1987 Bulletin 1987/15**

(73) Proprietor: **Chugai Seiyaku Kabushiki Kaisha Tokyo (JP)**

(72) Inventors:
 • **Tamura, Masahiko**
  **Kawasaki-shi Kanagawa-ken (JP)**
 • **Nomura, Hitoshi**
  **Tokyo (JP)**
 • **Hattori, Kunihiro**
  **Mitaka-shi Tokyo (JP)**
 • **Ono, Masayoshi**
  **Tokorozawa-shi Saitama-ken (JP)**

(74) Representative: **VOSSIUS & PARTNER**
**Postfach 86 07 67**
**81634 München (DE)**

(56) References cited:
 EP-A- 0 169 566     EP-A- 0 220 520
 EP-B- 237 545      WO-A-85/04188
 WO-A-86/03225      WO-A-86/04506
 WO-A-86/04605      GB-A- 2 016 477

 • CHEMICAL ABSTRACTS, vol. 82, no. 13, 31st March 1975, page 339, column 2, abstract no. 83974n, Columbus, Ohio, US; R.K. SHADDUCK "CSF [colony-stimulating factor] response to endotoxin in normal and lenkopenic recipients"
 • CHEMICAL ABSTRACTS, vol. 90, no. 7, 12th February 1979, page 439, column 2, abstract no. 52813v, Columbus, Ohio, US; B.L. ZIDAR et al.: "Serum colony-stimulating and colony-inhibitory activity in response to neutropenia"
 • Proc. Natl. Acad. Sci. USA, vol. 82, 1985, pp. 1526-1530; K. Welte et al
 • Exp. Hematol., vol. 9, April 1981, pp. 332-345; Francis et al
 • Acta Haematol. Jap., vol. 47, 1984, pp. 1714-1721; Motoyoshi
 • Nature, vol. 314, 1985, pp. 625-628; Nicola et al

EP 0 217 404 B2

**Description**

The present invention relates to the use of human granulocyte colony stimulating factor (hereinafter abbreviated to human G-CSF) for the preparation of a pharmaceutical composition for the treatment of neutropenia by increasing the number of neutrophil having a fully matured form.

When bone marrow cells as target cells and kidney cells or fetal cells were cultured by the double-layer soft agar cultivation method, with the bone marrow cells being in the upper layer and the kidney or fetal cells in the lower layer, part of the cells in the upper layer grew and differentiated to form colonies of neutrophilic granulocytes (hereunder simply referred to as granulocytes) or monocytic macrophages. This observation has led to the assumption of the presence in vivo of factors which promote the formation of colonies [Pluznik and Sach, J. Cell. Comp. Physiol., 66, 319 (1965); and Bradley and Metcalf, Aust. J. Exp. Biol. Med. Sci., 44, 287 (1966)].

These factors which are collectively referred to as CSF are known to be produced by cells, such as T-cells, monocytic macrophages, fibroblasts and endothelial cells, which normally are distributed extensively in vivo. Among subclasses of CSF are included: granulocyte-monocytic macrophage CSF (abbreviated as GM-CSF) which act on the stem cells of granulocytes or monocyte macrophages in such a manner that they stimulate the growth of such stem cells and induce their differentiation to form colonies of granulocytes or monocytic macrophages; monocytic macrophage CSF (abbreviated as M-CSF) which is principally capable of forming colonies of macrocytic macrophages; multipotent CSF (abbreviated as multi-CSF) which acts on less differentiated multipotent stem cells; and granulocyte CSF (abbreviated as G-CSF) of the type contemplated by the present invention which is principally capable of forming granulocytic colonies. It has recently been held that the stages of differentiation of target cells differ from one subclass of CSF to another [Asano, Taisha - Metabolism and Disease, 22, 249 (1985); and Yunis et al., "Growth and Maturation Factors", edited by Guroff, John Wiley & Sons, NY, vol. 1, 209 (1983)].

Many reports have been published on human CSF, in particular, CSF derived from normal human tissues and CSF derived from human tumor cells [see, for example, Stanley et al., Fed. Proc., 35, 2272 (1975); Burgess et al., Blood, 49, 573 (1977); Shah et al., Blood 50, 811 (1977); Fojo et al., Biochem., 17, 3109 (1978); Okabe et al., Cancer Res., 38, 3910 (1978); Asano et al., Blood, 49, 845 (1977); Golde et al., Blood 57, 1321 (1981); Wu et al, J. Biol. Chem., 254, 6226 (1979); and Dipersio et al., Blood, 56, 717 (1980)].

However, these species of human CSF are not in a completely pure form and much is left unknown about the utility or effectiveness of human CSF as a pharmaceutical agent.

The number of peripheral leukocytes present in a normal adult ranges from 4000 to 9000 per $mm^3$ [see Takigawa et al., Rinsho Kensa (Clinical Examination), 12, 12, 906 - 910, 1968], but for various reasons, their number can drop below 4000 and this abnormal state is generally referred to as leukopenia.

Leukopenia sometimes occurs as a result of uniform decrease in the number of various types of leukocytes but, more often than not, the disease is due to a decrease in the number of a particular type of leukocyte and may be classified as neutropenia, eosinopenia and lymphopenia according to the type of the leukocyte that has decreased in number. However, on a clinical basis, most cases of leukopenia are due to a decrease in the number of neutrophiles. The mechanism behind the decrease in the number of neutrophiles is two-fold: 1) lowered production of neutrophiles in the bone marrow; and 2) enhanced consumption and destruction of neutrophiles in the peripheral blood. While many causative diseases or lesions [such as essential ones that are caused by unknown factors and those which accompany exposure to radiation or a variety of blood diseases; for details, see Shindan to Chiryo (Diagnosis and Treatment), 70, 7, 24 - 31, 1982] are known to be responsible for these phenomena, most cases of neutropenia have occurred on account of the administration of various drugs and, in particular, the drug-induced decrease in the number of neutrophiles which can potentially lead to so-called agranulocytosis accompanied by a decrease in the absolute number of neutrophiles to 150/$mm^3$ or below [see, for example M. Komine, Ketsuekibyogaku (Hematology), p. 227 and p. 849, 1981] has clinically become a matter of great concern.

Many drugs can cause a decrease in the number of neutrophiles and are generally classified as two types: those which cause pancytopenia such as alkylating agents (e.g. cyclophosphamide), metabolic antagonists (cytosine arabinoside) and cancer-controlling antibiotics (e.g. daunorubicin); and those which reduce the number of neutrophiles by so-called drug hypersensitivity, such as sulfa agents, antipyretic analgesic sedatives (e.g. aminopyrine), anticonvulsants, antibiotics (e.g. chloramphenicol) and antithyroid agents [for details, see Shindan to Chiryo (Diagnosis and Treatment), 70,7, pp 24-31, 1981].

The number of neutrophilies can be increased by such drugs as cepharanthin, adenine agents, L-cystine, parotin and the decomposition products of ribonucleic acids. However, these drugs are not completely satisfactory for clinical purposes because of their low efffectiveness or because of the high incidence of side effects.

In addition, it is required that the neutrophiles which are restored or increasing in number should assume a sufficiently mature form to possess their inherent capabilities; otherwise, they would not be able to achieve the defense mechanism such as phagocytic action that is necessary for maintaining the normal state of a living organism.

Therefore, it has long been desired to develop a drug that is capable of increasing the number of neutrophiles having a fully mature form and which yet causes a reduced incidence of side effects.

To meet this demand, the assignee of the present invention established from the tumor cells of patients with oral cavity cancer a cell strain, CHU-1 (C.N.C.M. Accession Number 1-315), that had an extremely high capability of human G-CSF production and which exhibited a high capacity for growth. The assignee cultured this cell and successfully isolated from the supernatant of the culture a pure human G-CSF that displayed an activity for accelerating the formation of colonies of human neutrophiles (EP-A1 0 169 566). Subsequently, the assignee established a cell strain, CHU-2 (C.N.C.M. Accession Number I-483) from human oral cavity cancer, cultured this cell and also succeeded in isolating, from the supernatant of the culture, a G-CSF which was completely identical to the one derived from CHU-1.

The assignee continued their studies with a view to developing a method for G-CSF production by recombinant DNA technology that is capable of attaining a higher concentration of G-CSF than the previous methods of cell cultivation without requiring any complicated purifying procedures and, as a result, they succeeded in large-scale preparation of a homogenous human G-CSF by recombinant DNA technology (Japanese Patent Application Nos. 269455/1985, 269456/1985, 270838/1985 and 270839/1985).

On the basis of these accomplishments, the inventors of the present invention administered the above-described human G-CSF to normal animals and models that were suffering from a decrease in the number of neutrophiles. This factor caused a significant increase in the number of neutrophiles in the normal animals whereas it displayed an ability to inhibit to a significant extent the decrease in the number of neutrophiles in the neutropenic animal models. In addition, the increased neutrophiles assumed a fully matured form. Therefore, the present inventors found that the human G-CSF could be used as an effective penia treating agent. The present invention has been accomplished on the basis of this finding.

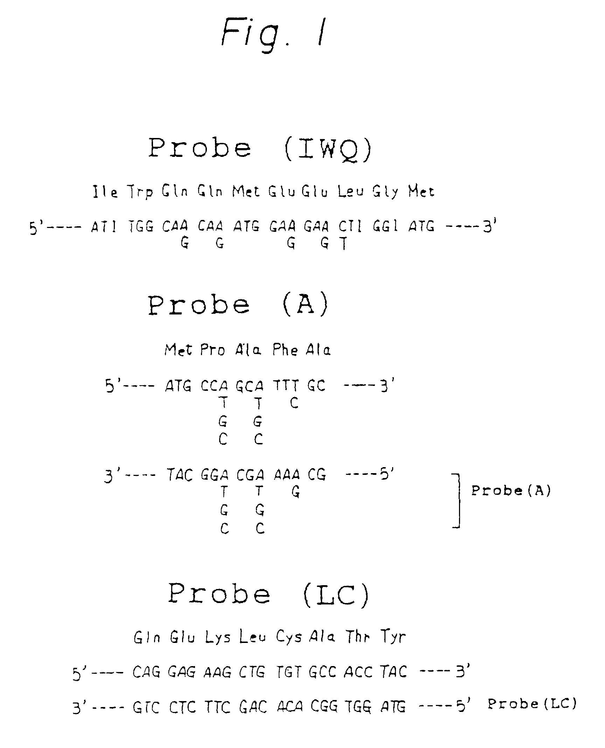

Fig. 1 shows the amino acid and the nucleotide sequences of three different probes, IWQ, A and LC;
Fig. 2 shows the nucleotide sequence of a cDNA insert in pBRG4;
Fig. 3 shows the nucleotide sequence of a cDNA insert in pBRV2;
Fig. 4 is a presentation of the process for constructing an expression recombinant vector pTN-G4:
Fig. 5 shows a process for constructing an expression recombinant vector pHGG4-dhfr;
Fig. 6 is a presentation of the process for constructing an expression recombinant vector pTN-V2:
Fig. 7 shows a process for constructing an expression recombinant vector pHGV2-dhfr.

Thus, the present invention relates to the embodiments characterized in the claims.

Any human G-CSF that has been purified to an extent that makes it suitable for use in a neutropenia treating agent may be employed as the active ingredient of the neutropenia treating agent of the present invention. Preferable human G-CSFs are ones obtained by isolation from the supernatant of the culture human G-CSF producing cell, and a polypeptide or glycoprotein having the human G-CSF activity that is obtained by transforming a host with a recombinant vector having incorporated therein a gene coding for a polypeptide having the human G-CSF activity.

Two particularly preferably examples of human G-CSF are shown below:

(1) human G-CSF having the followiqng physicochemical properties:

i) molecular weight: about 19,000 ± 1,000 as measured by electrophoresis through a sodium dodecylsulfate - polyacrylamide gel;
ii) isoelectric point: having at least one of the three isoelectric points, pI = 5.5 ± 0.1, pI = 5.8 ± 0.1, and pI = 6.1 ± 0.1;
iii) ultraviolet absorption: having a maximum absorption at 280 nm and a minimum absorption at 250 nm;
iv) amino acid sequence of the 21 residues from N terminus: $H_2N$-Thr-Pro-Leu-Gly-Pro-Ala-Ser-Ser-Leu-Pro-Gln-Ser-Phe-Leu-Leu-Lys-Cys-Leu-Glu-Gln-Val-

(2) human G-CSF containing either a polypeptide having the human granulocyte stimulating factor activity which is represented by all or part of the amino acid sequence shown below, or a glycoprotein having both said polypeptide and a sugar chain portion:

```
(Met)ₙThr  Pro  Leu  Gly  Pro  Ala  Ser  Ser  Leu  Pro
Gln  Ser  Phe  Leu  Leu  Lys  Cys  Leu  Glu  Gln  Val
Arg  Lys  Ile  Gln  Gly  Asp  Gly  Ala  Ala  Leu  Gln
Glu  Lys  Leu  (Val  Ser  Glu)ₘCys  Ala  Thr  Tyr  Lys
Leu  Cys  His  Pro  Glu  Glu  Leu  Val  Leu  Leu  Gly
His  Ser  Leu  Gly  Ile  Pro  Trp  Ala  Pro  Leu  Ser
Ser  Cys  Pro  Ser  Gln  Ala  Leu  Gln  Leu  Ala  Gly
Cys  Leu  Ser  Gln  Leu  His  Ser  Gly  Leu  Phe  Leu
Tyr  Gln  Gly  Leu  Leu  Gln  Ala  Leu  Glu  Gly  Ile
Ser  Pro  Glu  Leu  Gly  Pro  Thr  Leu  Asp  Thr  Leu
Gln  Leu  Asp  Val  Ala  Asp  Phe  Ala  Thr  Thr  Ile
Trp  Gln  Gln  Met  Glu  Glu  Leu  Gly  Met  Ala  Pro
Ala  Leu  Gln  Pro  Thr  Gln  Gly  Ala  Met  Pro  Ala
Phe  Ala  Ser  Ala  Phe  Gln  Arg  Arg  Ala  Gly  Gly
Val  Leu  Val  Ala  Ser  His  Leu  Gln  Ser  Phe  Leu
Glu  Val  Ser  Tyr  Arg  Val  Leu  Arg  His  Leu  Ala
Gln  Pro
```

(provided that m is 0 or 1; and n is 0 or 1).

The human G-CSFs (1) and (2) may be produced by the methods described in the Referential Examples shown later in this specification. Stated more specifically, human G-CSF (1) can be prepared by the methods described in Referential Examples 1 and 2, human G-CSF (2) by the methods described in Referential Examples 4 to 11.

For details of the conditions employed for performing these methods, see the specification of Japanese Patent Application Nos. 153273/1984, 269455/1985, 269456/1985, 270838/1985 and 270839/1985, all having been filed by the assignee of the present invention.

Another method that can be employed consists of performing fusion of a G-CSF producing cell with a self-proliferating malignant tumor cell and cultivating the resulting hybridoma in the presence or absence of mitogen.

All of the human G-CSFs that are prepared by the methods described above are included within the scope of the present invention.

The human G-CSF containing solution obtained may be stored in a frozen state after being further purified and concentrated, as required, by any known technique. Alternatively, the solution may be stored after being dehydrated by such means as freeze-drying or vacuum drying. If desired, the human G-CSF may be dissolved in an appropriate buffer, followed by aseptic filtration through a Millipore filter or any other suitable means so as to formulate an injection.

The neuropenia treating agent of the present invention may contain a pharmaceutical carrier or excipient necessary to assist in its formulation in a dosage form suitable for administration to humans or animals. If desired, a stabilizer and an anti-adsorption agent may also be incorporated in the agent.

The level of dosage and the frequency of administration of the human G-CSF in the neuropenia treating agent of the present invention may be determined in consideration of the severity of the disease to be treated: typically, a dosage containing 0.01 - 500 µg, preferably 0.5 - 200 µg, of human G-CSF may be administered to an adult in one to seven portions over a week. However, it should be noted that the present invention is by no means limited with respect to the content of human G-CSF.

Examples

The following referential examples and working examples are provided for the purpose of further illustrating the present invention but should in no way be taken as limiting.

Referential Example 1: Assaying CSF Activity

The following methods were used to determine the CSF activity (hereunder abbreviated as CSA) in the present invention.

CSA assay

(a) With human bone marrow cells:

Single-layer soft agar cultivation was conducted in accordance with the method of Bradley, T.R. and Metcalf, D. (Aust. J. Exp. Biol. Med. Sci., 44, 287-300, 1966). More specifically, 0.2 ml of a bovine fetal serum, 0.1 ml of the sample, 0.1 ml of a human bone marrow nonadherent cell suspension (1 - 2 x $10^5$ nuclear cells), 0.2 ml of a modified McCoy's 5A culture solution, and 0.4 ml of a modified McCoy's 5A culture solution containing 0.75% of agar were mixed, poured into a plastic dish for tissue culture (35 mm$^\emptyset$), coagulated, and cultured at 37° C in 5% $CO_2$/95% air and at 100% humidity. Ten days later, the number of colonies formed was counted (one colony consisting of at least 50 cells) and CSA was determined with one unit being the activity required for forming one colony.

(b) With mouse bone marrow cells:

A horse serum (0.4 ml), 0.1 ml of the sample, 0.1 ml of a C3H/He (female) mouse bone marrow cell suspension (0.5 - 1 x $10^5$ nuclear cells), and 0.4 ml of a modified McCoy's 5A culture solution containing 0.75% of agar were mixed, poured into a plastic dish for tissue culture (35 mm$^\emptyset$), coagulated, and cultured for 5 days at 37° C in 5% $CO_2$/95% air and at 100% humidity. The number of colonies formed was counted (one colony consisting of at least 50 cells) and CSA was determined with one unit being the activity for forming one colony.

The modified McCoy's 5A culture solution used in each of the methods (a) and (b) and the human bone marrow nonadherent cell suspension used in (a) were prepared by the following procedures.

Modified McCoy's 5A culture solution (double concentration)

Twelve grams of McCoy's 5A culture solution (Gibco), 2.55 g of MEM amino acid-vitamin medium (Nissui Seiyaku Co., Ltd.), 2.18 g of sodium bicarbonate and 50.000 units of potassium penicillin G were dissolved twice in 500 ml of distilled water and the solution was aseptically filtered through a Millipore filter (0.22 μm).

Human bone marrow nonadherent cell suspension

A bone marrow fluid obtained from a healthy person by sternal puncture was diluted 5-fold with an RPMI 1640 culture solution, plated over a Ficoll-Paque solution (Pharmacia Fine Chemicals) and centrifuged at 400 x g for 30 minutes at 25° C. The interfacial cell layer (specific gravity <1.077) was recovered. The cells were washed, adjusted to a concentration. of 5 x $10^6$ cells/ml with an RPMI 1640 culture solution containing 20% of bovine fetal serum, poured into a 25-cm$^2$ plastic flask for tissue culture, and incubated for 30 minutes in a $CO_2$ incubator. Nonadherent cells were recovered in the supernatant, poured into a plastic flask (25 cm$^2$) and incubated for 2 hours and a half. Nonadherent cells in the supernatant were collected and used in an assay.

Referential Example 2: Isolation of Human G-CSF

(1) Establishment of CHU-2

A tumor of a patient with oral cavity cancer wherein pronounced increase was observed in the number of neutrophiles was transplanted into nu/nu mice. About 10 days after the transplantation, the increase in the weight of the tumor and in the number of neutrophiles was significant. Twelve days after the transplantation, the tumor was extracted aseptically, dissected into cubes of 1 - 2 mm$^3$ and cultured in the following manner.

Ten to fifteen cubes of the tumor were put into a 50-ml plastic centrifugal tube. After addition of 5 ml of a trypsin solution (containing 0.25% of trypsin and 0.02% of EDTA), the tube was shaken for 10 minutes in a warm bath at 37°C and the supernatant was discarded. Another 5 ml of the same trypsin solution was added and trypsin digestion was conducted under agitation for 15 minutes at 37° C. The supernatant cell suspension was recovered and stored in ice after the trypsin had been inactivated by addition of 1 ml of a bovine fetal serum.

After repeating these procedures once again, the cell suspension was recovered, combined with the previously obtained suspension, and centrifuged at 400 x g for 10 minutes to obtain a cell pellet. The pellet was washed twice with F-10 containing 10% of a bovine fetal serum and was thereafter loaded in a plastic culture flask (25 cm$^2$) to give a cell concentration of 5 x 10$^6$ cells/flask. After incubation overnight in a $CO_2$ incubator (5% $CO_2$ and 100% humidity) with an F-10 culture solution containing 10% of a bovine fetal serum, the supernatant was removed together with the nonadherent cells, and culture was continued with a fresh supply of culture solution. Six days after the start of culture, the flask became full of the cells and the culture solution was replaced by a fresh one. On the next day, the culture solution was discarded and the flask was charged with 2 ml of an anti-mouse erythrocyte antibody (Cappel) diluted 5-fold with RPMI 1640 and 2 ml of a guinea pig complement (Kyokuto Seiyaku Co., Ltd.) diluted 2.5-fold with RPMI 1640. After incubation for 20 minutes at 37° C, the culture was washed twice with F-10 containing 10% of a bovine fetal serum and the nu/nu mouse derived fibroblasts were removed. Subsequently, an F-10 culture solution containing 10% of a bovine fetal serum was added and cultivation was conducted for 2 more days. Thereafter, same of the cells were recovered and subjected to cloning by the limiting dilution method.

The resulting 11 clones were checked for their CSF activity and one clone (CHU-2) exhibited activity about 10 times as high as that of the other clones.

(2) Isolation of Human G-CSF

The cells established in (1) were grown in a completely dense population (grown to confluency) in two culture flasks (150 cm$^2$). The cells were recovered, suspended in 500 ml of an F-10 culture solution containing 10% of a bovine fetal serum, transferred into a glass roller bottle of 1580 cm$^2$ (Belco), and whirl-cultured at 0.5 rpm. When the cells were found to have grown in a completely dense population (grown to confluency) on the inner wall of the roller bottle, the culture solution was replaced by a serum-free RPMI 1640. After 4-day culture, the supernatant of the culture was recovered and cultivation was continued with F-10 containing 10% of a bovine fetal serum being added. After 3-day culture, the culture solution was again replaced by a serum-free RPMI 1640 and the supernatant of the culture was recovered 4 days later. By repeating these procedures, 500 ml of the serum-free supernatant of culture per bottle was obtained each week. In addition, this method enabled the supernatant of culture to be recovered, with the cells maintained over a significantly prolonged period.

A batch consisting of 5,000 ml of the supernatant of the culture obtained was mixed with 0.01% of Tween 20 and concentrated about 1000 times by ultrafiltration with Hollow Fiber DC-4 and Amicon PM-10 (Amicon). The concentrate was purified by the following steps.

(i) A portion (5 ml) of the concentrated supernatant of culture was subjected to get filtration on an Ultrogel AcA54 column (4.6 cm$^\varnothing$ x 90 cm$^L$; LKB) at a flow rate of ca. 50 ml/hr with 0.01 M Tris-HCl buffer (pH 7.4) containing .015 M NaCl and 0.01% Tween 20 (Nakai Kagaku Co., Ltd.) The column had been calibrated with bovine serum albumin (Mw: 67,000), ovoalbumin (Mw; 45,000) and cytochrome C (Mw; 12,400). After completion of the get filtration, 0.1 ml of each of the fractions was diluted 10-told and screened for the active fractions by the above-described method of CSA assay (b). The fractions for Ve = 400 - 700 ml were found to exhibit macrophage-dominant CSA while the fractions for Ve = 800 - 1200 ml showed granulocyte-dominant CSA. Therefore, the latter fractions were collected and concentrated to a volume of ca. 5 ml on an ultrafiltration apparatus with PM-10 (Amicon).

(ii) To the cocentrated fractions was added an aqueous solution of 0.1% trifluoroacetic acid containing 30% of n-propanol (for determination of amino acid sequence; available from Tokyo Kasei K.K.) After the mixture had been left to stand in ice for about 15 minutes, the precipitate was removed by centrifugation for 10 minutes at 27,000 x g. The supernatant was adsorbed on a μ-Bondapak C18 column (8 mm x 30 cm for semipreparatory use; Waters) equilibrated with the aqueous solution containing n-propanol and trifluoroacetic acid; the column was continuously eluted with an aqueous solution of 0.1% trifluoroacetic acid which contained n-propanol having a linear concentration gradient of 30 - 60%. A high performance liquid chromatographic apparatus, Hitachi Model 685-50 (Hitachi, Ltd.), and a detector, Hitachi Model 638-41 (Hitachi, Ltd.) were employed to determine the absorptions at 220 nm and 280 nm simultaneously. After elution, 10 μl of each of the fractions was diluted 100-fold and screened for the active fractions by the above-described method of CSA assay (b). The peaks eluted with 40% n-propanol were found to have CSA activity, so they were collected, re-chromatographed under the same conditions, and assayed for CSA by the same method. Again, CSA activity was observed in the peaks at 40% n-propanol. Therefore, these peaks were collected (4 fractions = 4 ml) and freeze-dried.

(iii) The freeze-dried powder was dissolved in 200 μl of an aqueous solution of 0.1% trifluoroacetic acid containing 40% of n-propanol, and the solution was subjected to high performance liquid chromotography on TSK-G 3000SW column (Toyo Soda Manufacturing Co., Ltd.; 7.5 mm x 60 cm). Elution was conducted with the same aqueous solution at a flow rate of 0.4 ml/min and the fractions were taken in 0.4-ml portions with a fraction collector. FRAC-100 (Pharmacia Fine Chemicals). Each of the fractions taken was checked for its CSA by the same method as described above and activity was observed in the fractions for retention times of 37 - 38 minutes (corresponding to MW of ca. 2 x 10$^4$). The active fractions were recovered and purified on an analytical μ-Bondapak C18 column

(4.6 mm x 30 cm). The main peaks were recovered and freeze-dried. The sample obtained was assayed by the method of CSA assay (a); it was found to have human G-CSF activity.

Referential Example 3: Physicochemical Properties

The physicochemical properties of human G-CSF which was prepared in Referential Example 2 were determined by the following analyses and test.

(i) Molecular weight

The molecular weight of the CSF was determined by sodium dodecylsulfate-polyacrylamide gel electrophoresis (SDS-PAGE). The electrophoretic equipment was PROTEAN$^{TH}$ (16 cm, product of Bio-Rad Corporation), using a get made up of a polyacrylamide slab get (T = 15%, C = 2.6%) measuring 140 mm x 160 mm x 1.5 mm, and a concentrating get (T = 3%, C = 20%). A denatured CSF sample was prepared by the following procedure: CSF was boiled for 3 minutes in a solution containing 2% of sodium dodecylsulfate in 0.46 M 2-mercaptoethanol. After performing electrophoresis with 4 $\mu$g of the sample with a constant current of 30 mA for 4 hours, the get was removed and stained with 0.25% Coomassie Brilliant Blue R 250 (product of Sigma Chemical Co.) for band detection. The following substances were used as molecular weight markers after similar treatments: phosphorylase B (mol. wt. 92.500), bovine serum albumin (BSA, mol. wt. 67,000), ovalbumin (OVA, mol. wt. 45,000), carbonic anhydrase (mol. wt. 31,000), soybean trypsin inhibitor (mol. wt. 21,500) and lysozyme (mol wt. 14,400). A single band corresponding to a molecular weight of approximately 19.000 was detected.

In view of the result, the human G-CSF of the present invention is deemed to have a molecular weight of 19,000 $\pm$ 1,000.

(ii) Isoelectric point

The isoelectric point of the CSF of the present invention was determined by a flat bed, isoelectric electrophoretic apparatus, FBE-3000 (product of Pharmacia Fine Chemicals). After 2-hr electrophoresis with a constant power of 30 watts (Vmax = 2,000 volts) on a polyacrylamide get (T = 5%, C = 3%, 115 mm x 230 mm) containing Pharmalyte (pH = 4 - 6.5, Pharmacia Fine Chemicals) and 4M urea, the CSF was fixed with 30% methanol/10% trichloroacetic acid/ 35% sulfosalicylic acid, and stained with Coomassy Brilliant Blue R-250. A Low pl kit (pH: 2.5 - 6.5, product of Pharmacia Fine Chemicals) was used as an isoelectric point marker.

Analysis of band separation in the pH range of 4 to 6.5 gave three distinct bands corresponding to pl = 5.52, 5,80 and 6.13, among which the two bands for pl = 5.52 and 5,80 were predominant components.

Another five measurements of the same sample of CSF gave the following results: pl = 5.5 $\pm$ 0.1, 5.8 $\pm$ 0.1 and 6.1 $\pm$ 0.1.

(iii) UV absorption

The sample was checked for its UV absorption with a spectrophotometer, with 0.1% trifluoroacetic acid containing 40% of n-propanol being taken as a reference. A maximum peak occurred at 280 nm and a minimum peak at 250 nm.

(iv) Determination of amino acid sequence

① Determination of N-terminal amino acid sequence

The sample was subjected to Edman degradation with a gas-phase sequencer (Applied Biosystems) and the resulting PTH amino acid was analyzed by routine procedures with a high performance liquid chromatographic apparatus (Beckman Instruments) and Ultrasphere-ODS column (Beckman Instruments). The column (5 $\mu$m; 4.6 mm$^{\varnothing}$ x 250 mm$^L$) was equilibrated with a starting buffer [aq. sol. containing 15 mM sodium acetate buffer (pH 4.5) and 40% acetonitrile] and injected with the sample (as dissolved in 20 $\mu$l of the starting buffer). Separation was effected by isocratic elution with the starting buffer. The flow rate was 1.4 ml/min and the column temperature was held at 40° C. Detection of the PTH amino acid was achieved utilizing the absorptions in the UV range at 269 nm and 320 nm. Standard samples of PTH amino acid (Sigma) in 2-nmol portions were separated on the same line to determine their retention times, which were compared with those of the sample to be tested. As a result, the sample was found to have the following amino acid sequence of the 40 residues from N-terminus:

```
H2N - Thr - Pro - Leu - Gly - Pro - Ala - Ser - Ser -
           (10)
Leu - Pro - Gln - Ser - Phe - Leu - Leu - Lys - Cys -
           (20)
Leu - Glu - Gln - Val - Arg - Lys - Ile - Gln - Gly -
                 (30)
Asp - Gly - Ala - Ala - Leu - Gln - Glu - Lys - Leu -
                       (40)
Cys - Ala - Thr - Tyr - Lys -
```

② Degradation with bromocyan

The sample was dissolved in 70% formic acid. To the solution, 200 equivalent amounts of bromocyan that had been purified by sublimation was added. The mixture was left overnight at 37°C for reaction. The reaction product was freeze-dried and fractionated by HPLC on a TSK G3000SW column (Toyo Soda Manufacturing Co., Ltd.) to obtain four peaks. The peaks were named CN-1, CN-2, CN-3 and CN-4 in the decreasing order of the molecular weight. The first two peaks (CN-1 and CN-2) had better yields and their amino acid sequences were analyzed with an automatic gas-phase sequencer (Applied Biosystems) under the same conditions as used in ①.

As a result, CN-1 was found to be a peptide from the N-terminus of G-CSF protein, and CN-2 had the following amino acid sequence:

```
Pro - Ala - Phe - Ala - Ser - Ala - Phe -

Gln - Arg - Arg - Ala - Gly - Gly - Val -

Leu - Val - Ala - Ser - His - Leu - Gln -
```

③ Digestion with trypsin

The sample was dissolved in 0.1 M Tris-HCl buffer (pH 7.4) containing 8 M urea and the solution was mixed with 0.1 M Tris-HCl buffer (pH 7.4) containing 0.1% 2-mercaptoethanol to provide a final urea concentration of 2 M. A TPCK-treated trypsin (Sigma) was added such that the sample-to-enzyme ratio was 50:1. The mixture was held for 4 hours at 25° C and, after addition of an equal amount of TPCK-treated trypsin, the mixture was held for an additional 16 hours at 25° C. Thereafter, the reaction product was subjected to high-speed reverse-phased column chromatography on C8 column (Yamamura Kagaku K-K), with elution conducted with 0.1% TFA containing n-propanol having a linear density gradient of 5 - 60%. While several peaks were obtained by measuring the absorption at 280 nm, the main peak was analyzed for its amino acid sequence with an automatic gas-phase sequencer (Applied Biosystems) under the same conditions as used in ①. As a result, the main peak was found to be a peptide having the following sequence which contained part of the CN-2 fragment shown in ②:

```
Gln - Leu - Asp - Val - Ala - Asp - Phe - Ala - Thr -

Thr - Ile - Trp - Gln - Gln - Met - Glu - Glu - Leu -

Gly - Met - Ala - Pro - Ala - Leu - Gln - Pro - Thr -

Gln - Gly - Ala - Met - Pro - Ala - Phe - Ala - Ser -
```

(v) Amino acids in the protein portion

The sample was hydrolyzed by a conventional method and analyzed for the amino acid composition of the protein portion with an automatic amino acid analyzer, Model 835 of Hitachi, Ltd. The results are shown in Table 1. The hydrolysis was conducted under the following conditions:

① 6 N HCl, 110° C, 24 hours, in vacuum
② 4 N methanesulfonic acid + 0.2% 3-(2-aminoethyl)indole, 110° C, 24 hours, 48 hours, 72 hours, in vacuum

The sample was dissolved in a solution (1.5 ml) containing 40% n-propanol and 0.1% trifluoroacetic acid. Aliquots each weighing 0.1 ml were dried with a dry nitrogen gas and, after addition of the reagents listed in ① or ②, the containers were sealed in vacuum followed by hydrolysis of the contents.

Each of the values shown in Table 1 was the average of four measurements, 24 hour value for ① and 24, 48 and 72 hour values for ② except that the contents of Thr, Ser, 1/2 Cys, Met, Val, Ile and Trp were calculated by the following methods [see "Tampaku Kagaku (Protein Chemistry) II", A Course in Biochemical Experiments, Tokyo Kagaku Dohjin]:

-- For Thr, Ser, 1/2 Cys and Met, the time-dependent profile of the 24, 48 and 72 hour values for ② was extrapolated by zero hours.
-- For Val and Ile, the 72 hour value for ② was used.
-- For Trp, the average of 24, 48 and 72 hour values for ② was used.

The values shown in the column of "Predicted Number of Residual Amino Acid Groups" are based on the assumption of the existence of 33 Leu's. Generally, the amino acids that require correction of the type described above are decomposed either somewhat or substantially during hydrolysis or they are resistant to hydrolysis. In particular. Pro Produces a low color yield. Primarily for these reasons, the actually measured contents (nmol) of amino acids of interest and hence, the calculated number of each of the residues, has a tendency to be lower than theoretical values (see Seikagaku Jikken Koza, ibid).

Table 1

| Amino acids | Measured values (nmol) | Predicted number of amino acid residues (rounded to the parenthesized integrals) |
|---|---|---|
| Asp (Asp + Asn) | 3.54 | 4.3 (4) |
| Thr | 4.58 | 5.5 (6) |
| Ser | 10.64 | 12.9 (13) |
| Glu (Glu + Gln) | 22.31 | 27.0 (27) |
| Pro | 8.30 | 10.1 (10) |
| Gly | 10.60 | 12.8 (13) |
| Ala | 14.85 | 18.0 (18) |
| 1/2 Cys | 2.59 | 3.1 (3) |
| Val | 6.16 | 7.5 (7) |
| Met | 2.26 | 2.7 (3) |
| Ile | 3.29 | 4.0 (4) |
| Leu | 27.24 | 33.0 (33) |
| Tyr | 2.60 | 3.1 (3) |
| Phe | 5.08 | 6.1 (6) |
| Lys | 3.68 | 4.5 (4) |
| His | 3.93 | 4.8 (5) |
| Trp | 1.61 | 1.9 (2) |
| Arg | 4.29 | 5.2 (5) |
| Total | | (166) |
| Calculated molecular weight (no sugar counted in 166 residues) | | 17961 |

Referential Example 4: Probe Preparation

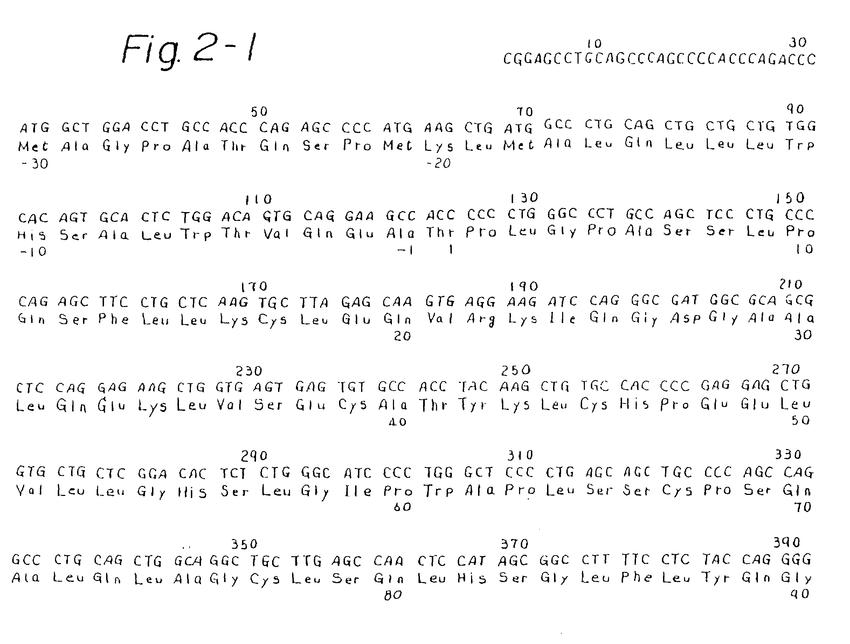

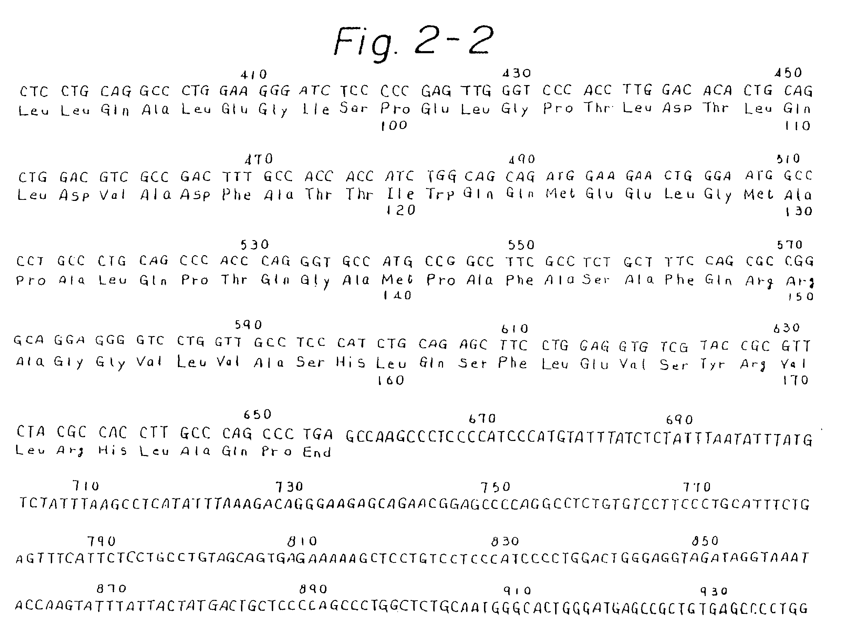

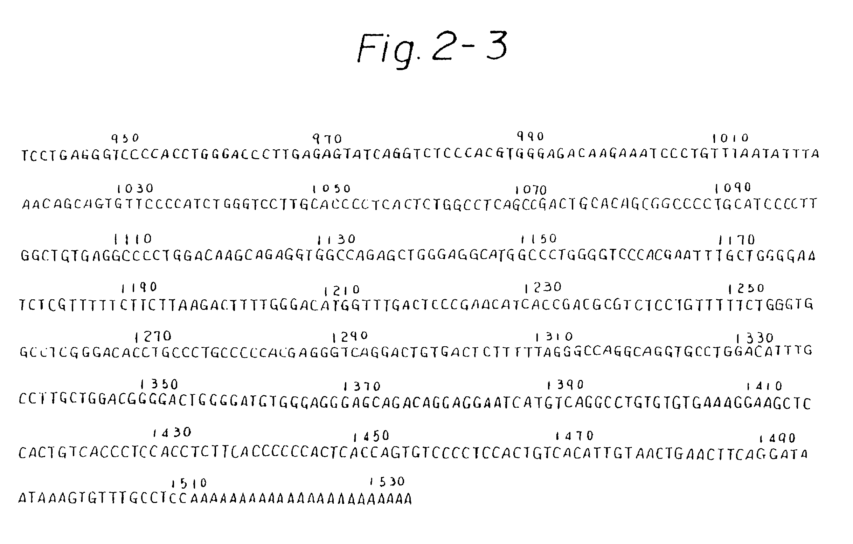

Based on the amino acid sequences determined in Referential Example 3 (iv), three nucleotide probes. (A). (LC) and (IWQ) were synthesized. Probe (A) was of the mixed type composed of 14 successive nucleotides. Probe (IWQ) was composed of 30 successive nucleotides with deoxyinosine and was a probe of the type used in the cloning of the human cholecystokinin gene [Takahashi et al., Proc. Natl. Acad. Sci., USA, 82, 1931 (1985)]. Probe (LC) was a 24-nucleotide probe that was synthesized on the basis of the nucleotide sequence shown in Fig. 2; it had a nucleotide sequence which corresponds to the nucleotide sequence at position 32 to 39 from the N terminus of the amino acid sequence shown in Referential Example 3 (iv)①.

Chemical synthesis of nucleotides can be achieved by applying the improved phosphotriester method to the solid phase method and has been reviewed by Narang [Tetrahedron, 39, 3-22 (1983)].

Probes based on amino acid sequences at positions other than those in the above-mentioned probes may also be used.

Referential Example 5: Construction of cDNA Library

CHU-2 cells were homogenized after addition of a guanidine thiocyanate solution and the total RNA was obtained by CsCI density gradient centrifugation.

Poly (A+) RNA was isolated from the total RNA by column chromatography on oligo(dT)-cellulose. Thereafter, a single-stranded cDNA was synthesized with a reverse transcriptase, and RNase H and E. coli DNA polymerase I were added to obtain a double-stranded cDNA. A dC chain was attached to the obtained double-stranded cDNA, which was joined to a vector, pBR322, to which a dG chain had been attached at the Pst I cleavage site. The resulting recombinant DNA was used to transform a strain of E. coli, X1776, and a pBR322-line cDNA library was constructed.

In a similar manner, the double-stranded cDNA was joined to the λgt10 vector with the EcoRI linker and λ-phage line cDNA library was constructed.

Referential Example 6: Screening

Two line of cDNA were obtained by the following procedures.

Recombinants derived from the pBR322-line cDNA library were fixed on Whatmann 541 filter paper and a single clone could be selected by colony hybridization with $^{32}$p-labelled probe (IWQ). Further study with the Southern blotting method [Southern, J. Mol. Biol., 98, 503 (1975)] showed that this clone also hybridized with probe (A). The nucleotide sequence of this clone was determined by the dideoxy method [Sanger. Science, 214, 1205 (1981)].

The obtained cDNA insert was found to consist of 308 base pairs including probes (IWQ) and (A), and have an open reading frame coding for 83 amino acids containing the amino acid sequence shown in Referential Example 3 (iv)-③. The pBR322-derived plasmid containing these 308 base pairs is hereunder referred to as pHCS-1.

A DNA fragment containing the 308 base pairs obtained from pHCS-1 was radiolabelled by the nick translation method (see Molecular Cloning, ibid.) and, with this fragment used as a probe, the λgt10-derived cDNA library was screened by plaque hybridization [Benton and Davis, Science, 196, 180 (1977)] to obtain five clones. The nucleotide sequence of a clone which was believed to contain cDNA was determined by the same method as described above (Fig. 2).

As shown in Fig. 2, this cDNA insert had a single large open reading frame.

The amino acid sequence encoded by this cDNA can be deduced as shown in Fig. 2.

The cDNA of this line is hereinafter referred to as cDNA (+VSE).

Comparison with then N-terminal amino acid sequence of G-CSF protein shown in Example 3 (iv) revealed that this cDNA contained a nucleotide sequence which corresponded to both a signal peptide encoded by 90 base pairs starting with the ATG sequence at 32 - 34 nucleotide positions from 5'-terminus and ending with the GCC sequence at 119 - 121 positions, and a mature G-CSF polypeptide encoded by 531 base pairs starting with the ACC sequence at 122 - 124 positions and ending with the CCC sequence at 650 - 652 positions. Therefore, the precursor polypeptide which comprised the signal peptide and the mature G-CSF polypeptide was composed of 207 amino acids and its molecular weight was calculated as 22292.67 daltons. The mature G-CSF polypeptide was composed of 177 amino acids and its molecular weight was calculated as 18986.74 daltons.

It should be noted that the ATG at 32 - 34 positions or at 68 - 70 positions can also be considered to be the protein initiation site. Escherichia coli strain X1776 R-1 harboring pBR322 which had this cDNA (+VSE) at the EcoRI cleavage site has been deposited with the Fermentation Research Institute, the Agency of Industrial Science and Technology (FERM BP-954).

This cDNA was joined to pBR327 [Soberon et al., Gene, 9, 287 (1980)] at the EcoRI site and the resulting plasmid is hereunder referred to as pBRG4.

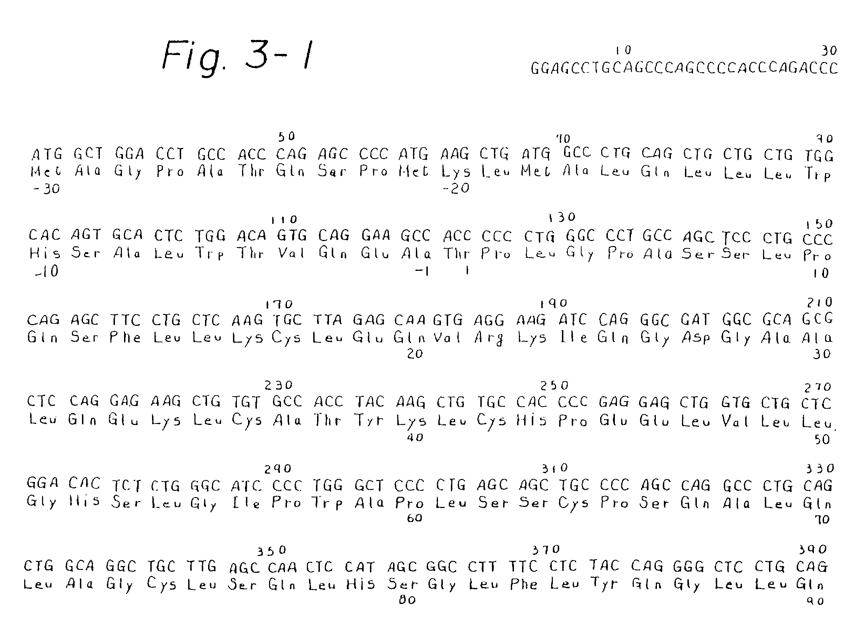

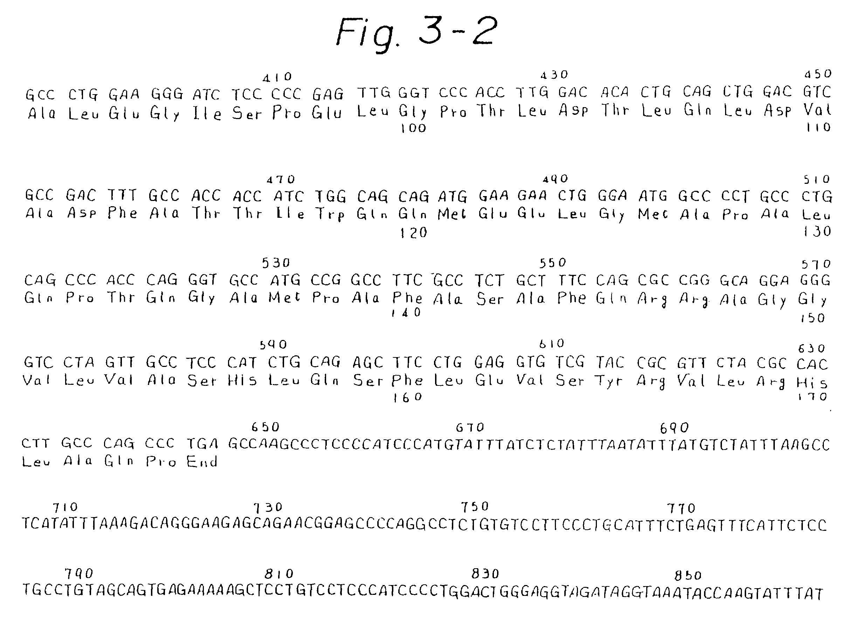

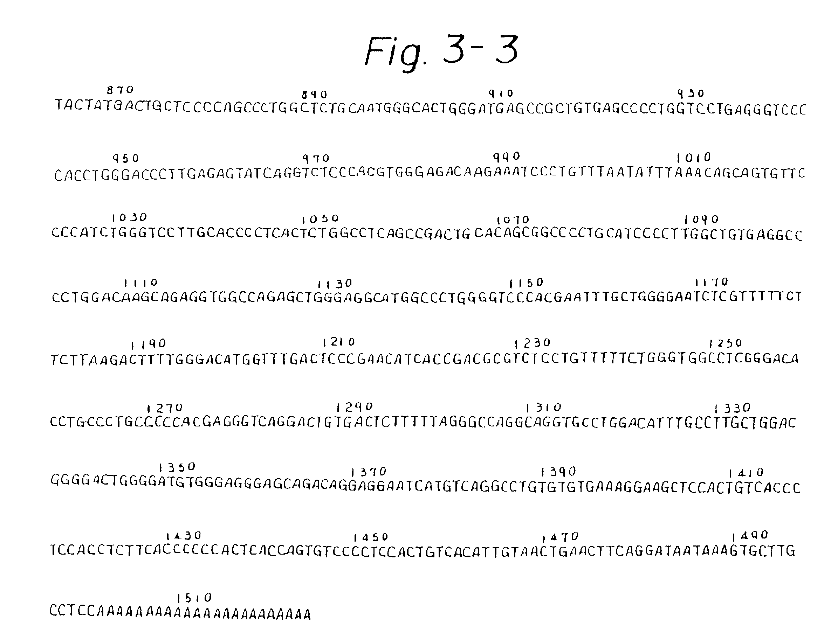

The thus obtained pBRG4 ws treated with a restriction enzyme, EcoRI, to obtain a DNA fragment containing cDNA of about 1500 base pairs. This fragment was radiolabelled by the nick translation method (see Molecular Cloning, ibid.) and, with this radiolabelled DNA fragment being used as a probe, the λgt10-derived cDNA library was screened once again by plaque hybridization (see Benton and Davis, ibid.) In this plaque hybridization, two sheets of λ-phage DNA fixed nitrocellulose filter paper were prepared: on of these sheets was used for the above-mentioned plaque hybridization and another one was subjected to plaque hybridization with the already described probe (LC). The phages which turned positive for both probes were selected. A clone which has a "full-length" cDNA was selected and the nucleotide sequence of the cDNA insert as determined by the dideoxy method is shown in Fig. 3.

This cDNA had a single large open reading frame and the amino acid sequence that would be encoded by this cDNA was deduced as shown in Fig. 3.

The cDNA of this line is hereinafter referred to as cDNA (-VSE).

Comparison with the N-terminal amino acid sequence of G-CSF protein shown in Example 3 (iv)-① revealed that this cDNA contained a nucleotide sequence which corresponded to both a signal peptide encoded by 90 base pairs starting with the ATG sequence at 31 - 33 nucleotide positions from 5'-terminus and ending with the GCC sequence at 118 - 120 positions, and a mature G-CSF polypeptide encoded by 522 base pairs starting with the ACC sequence at 121 - 123 positions and ending with the CCC sequence at 640 - 642 positions. Therefore, the precursor polypeptide which comprised the signal peptide and the mature G-CSF polypeptide was composed of 204 amino acids and its molecular weight was calculated as 21977.35 daltons. The mature G-CSF polypeptide was composed of 174 amino acids and its molecular weight was calculated as 18671.42 daltons.

It should be noted that ATG at 58 - 60 positions or at 67 - 69 positions can also be considered to be the protein initiation site.

Escherichia coli strain X1776 R-2 harboring pBR322 which had this cDNA (-VSE) at the EcoRI cleavage site has been deposited with the Fermentation Research Institute, the Agency of Industrial Science and Technology (FERM BP-955).

This cDNA was joined to pBR327 at the EcoRI site to form a plasmid which is hereunder referred to as pBRV2.

Referential Example 7: Construction of Recombinant Vector for Expression in E. coli

(A) +VSE line recombinant vector

From the pBRG4 plasmid obtained in Referential Example 6, a cDNA fragment of the G-CSF polypeptide was cut out with a restriction enzyme and a recombinant vector was constructed by one of the following methods:

(i) using an annealed synthetic linker, the fragment was ligated with a fragment prepared from a tac promoter-containing pKK223-3 (Pharmacia Fine Chemicals);
(ii) three fragments prepared from $P_L$ promoter containing pPL-lambda (Pharmacia Fine Chemicals) were ligated with an annealed synthetic linker and, the ligation product and the cDNA fragment were subjected to re-preparation procedures to construct a recombinant vector; or
(iii) using an annealed synthetic linker, the fragment was ligated with a fragment prepared from a trp promoter-containing pOYI plasmid.

(B) -VSE line recombinant vector

In the same manner as described above, three recombinant vectors were constructed using the plasmid pBRV2 obtained in Referential Example 6.

Referential Example 8: Preparation of E. coli Transformants, and Cultivation and Expression Thereof

Using three recombinant vectors of each of the +VSE and -VSE lines, E. coli strain DH1, N4830 or JM105 was transformed by the calcium chloride or rubidium chloride procedure described in Molecular Cloning, ibid. Each of the transformants obtained was cultivated in ampicillin-containing Luria medium, with induction being subsequently conducted as required to achieve expression.

Referential Example 9: Recovery and Purification of G-CSF Polypeptide from E. coli and Amino Acid Analysis Thereof

A culture solution of the transformants was centrifuged to obtain a cell pellet. The collected cells were treated with lysozyme and, after lysis by cyclic freezing and thawing, the supernatant was obtained. Alternatively, the cells were treated with guanidium chloride, centrifuged and the supernatant was recovered.

The supernatant was subjected to get filtration on an Ultrogel ACA54 column (LKB) and the active fractions were concentrated with an ultrafiltration apparatus.

Subsequently, an aqueous solution of trifluoroacetic acid containing n-propanol was added to the concentrate and, after being left in ice, the mixture was centrifuged and adsorbed on a reverse-phase C18 column. After elution, the fractions were checked for their activity. The active fractions were collected and subjected to the same procedures of purification as described above. The purified fractions were freeze-dried and the powder was dissolved and subjected to high performance liquid chromatography based on molecular size. The obtained polypeptides were subjected to SDS-polyacrylamide get electrophoresis and a single band for the desired G-CSF polypeptide was confirmed. The so obtained polypeptide showed human G-CSF activity. The G-CSF polypeptide was analyzed by an amino acid analyzing method with a Hitachi 835 Automatic Amino Acid Analyzer (Hitachi, Ltd.) For analysis of the N-terminal amino acids, a gas-phase sequencer (for Edman decomposition), high performance liquid chromatographic apparatus and Ultrasphere-ODS column were used.

Referential Example 10: Construction of Recombinant Vectors for Animal Cells

Recombinant vectors (derived from BPV) for use with C127 and NIH3T3 cells as host cells were constructed for each of the + VSE and -VSE line cDNAs. Recombinant vectors (with dhfr) for use with CHO cells were also constructed for each of th + VSE and -VSE line cDNAs. In the following, representative examples are described and, for further details, reference should be made to the relevant Japanese Patent Applications No. 269456/1986 and No. 270839/1986.

(A) Construction of recombinant vectors of the + VSE line

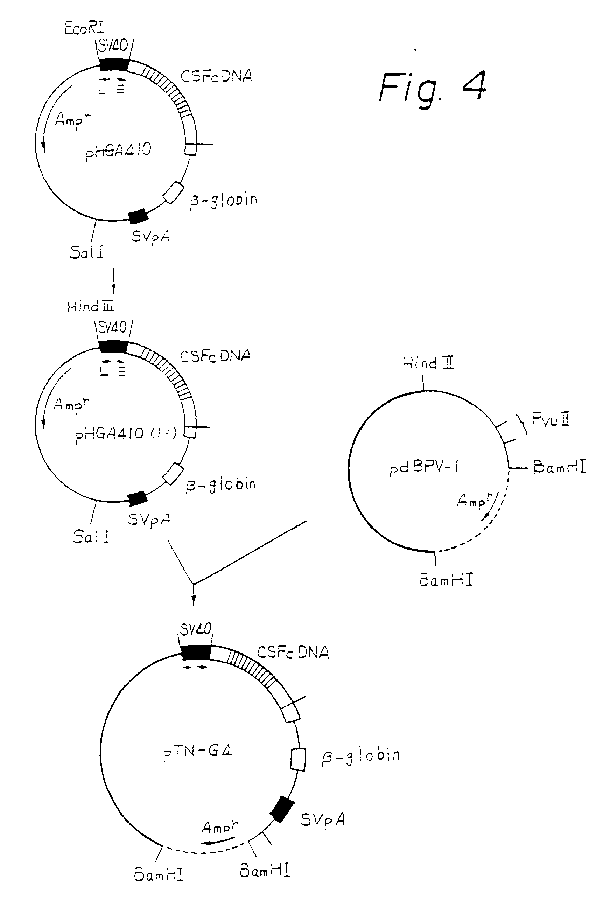

The cDNA (+VSE) fragment obtained in Referential Example 6 was inserted into a vector pdKCR to make a plasmid pHGA410, which was partially digested with EcoRI followed by treatment with DNA polymerase I (Kienow fragment) to create blunt ends. A linker HindIll was attached to the DNA, which was subsequently treated with HindIll and T4DNA ligase. The treated DNA was used to transform E. coli strain DH1 by the rubidium chloride procedure (see Molecular Cloning, ibid.) The resulting plasmid was named pHGA410(H) (Fig. 4).

The pHGA410(H) was treated with Sall and, after blunt ends were created, it was treated with HindIll once again and a HindIII-Sall fragment was recovered. A plasmid pdBPV-I having a transformed fragment of bovine papilloma virus was treated with HindIll and Pvull and the larger DNA fragment was separated and joined to the separately prepared HindIII-Sall fragment. The joined fragments were used to transform E. coli strain DH1 to obtain a plasmid, pTN-G4, which had the pHGA410-derived CSF-cDNA (Fig. 4).

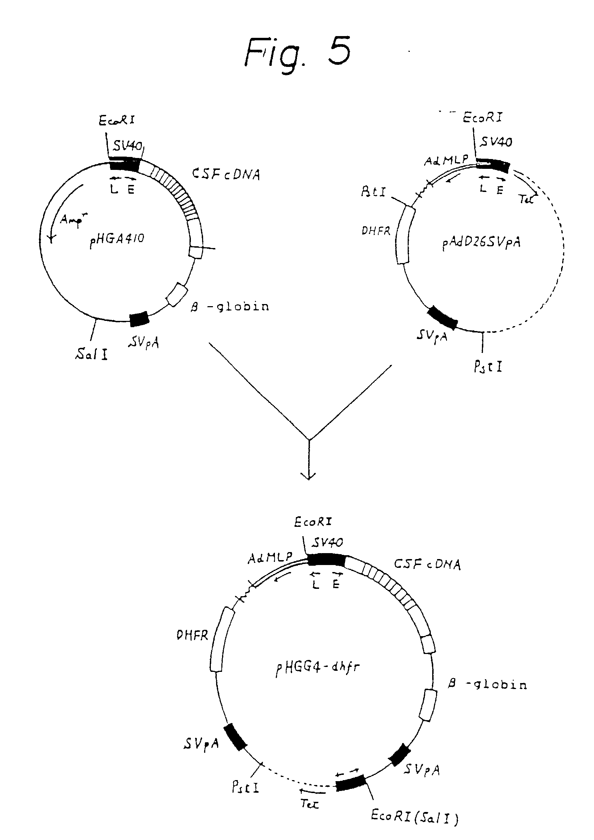

Either plasmid, pHGA410 or pHGA410(H), in combination with the plasmid pAdD26SVpA was used to construct pHGG4-dhfr which was a recombinant vector (+ VSE) for use with CHO cells (Fig. 5).

(B) construction of -VSE line recombinant vectors

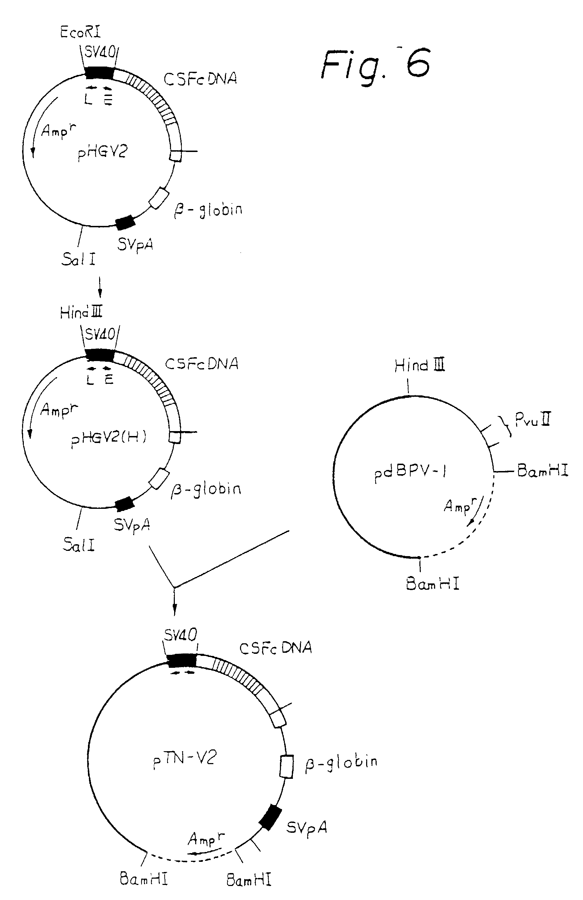

The cDNA (-VSE) fragment obtained in Referential Example 6 was inserted into a vector pdKCR to make a plasmid pHGV2, which was partially digested with EcoRI followed by treatment with polymerase I (Klenow fragment) to create blunt ends. A linker HindIll was attached to the DNA, which was subsequently treated with HindIll and T4DNA ligase. The treated DNA was used to transform E. coli strain DH1 by the rubidium chloride procedure (see Molecular Cloning, ibid.) The resulting plasmid was named pHGV2(H) (Fig. 6).

The pHGV2(H) was treated with Sall and, after blunt ends were created, it was treated with HindIll once again and a HindIII-Sall fragment was recovered. A plasmid pdBPV-1 having transformed fragment of bovine papilloma virus was treated with HindIll and PvulII and the larger DNA fragment was separated and joined to the separately prepared HindIII-Sall fragment. The joined fragments were used to transform E. coli strain DH1 to obtain a plasmid, pTN-V2, which had the pHGV2-derived CSF-cDNA (Fig. 6).

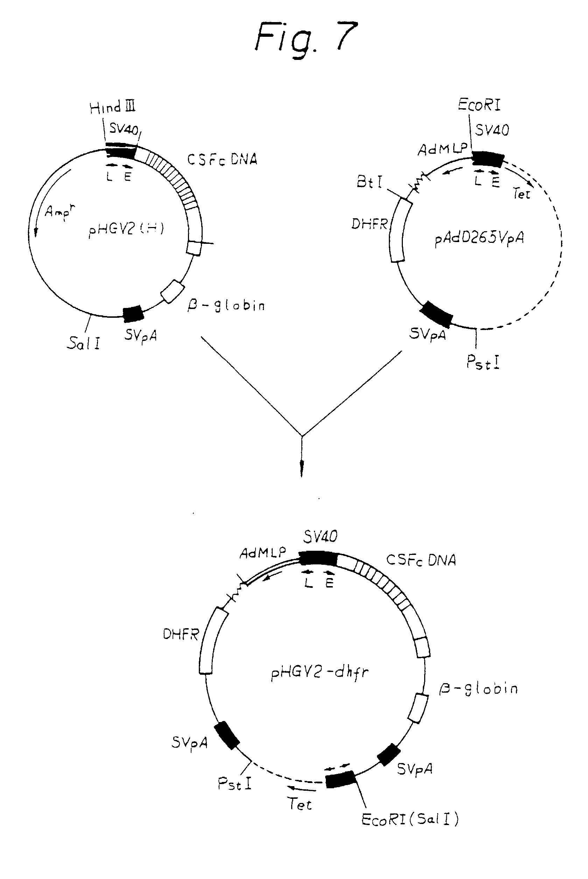

By similar procedures either plasmid, pHGV2 or pHGV2(H), in combination with the plasmid pAdD26SVpA was used to construct pHGV2-dhfr which was a recombinant vector (-VSE) for use with CHO cells (Fig. 7).

Referential Example 11: Transformation of Animal Cells and G-CSF Expression Therein

Two representative examples are hereunder described for (A) the transformation of mouse C127 cells and G-CSF (+VSE) expression therein; and (B) the transformation of CHO cells and G-CSF (-VSE) expression therein.

(A) Transformation of mouse C127 cells and G-CSF (+ VSE) expression therein

Before it was used to transform mouse C127 cells, the pTN-G4 obtained in Referential Example 10(A) was treated with a restriction enzyme, BamHI. Twenty micrograms of the plasmid pTN-G4 was dissolved in 100 $\mu$l of a reaction solution [10 mM Tris-HCl (pH 8.0), 7 mM $MgCl_2$, 100 mM NaCl, 2 mM 2-mercaptoethanol and 0.01% BSA] and treated with 20 units of BamHI (Takara Shuzo Co., Ltd.), followed by treatments with phenol and ether, and precipitation with ethanol.

Mouse C127 cells were grown in a Dulbecco's minimal essential medium containing 10% bovine fetal serum (Gibco). The C127 cells growing on plates (5 cm$^{\varnothing}$) were transformed with 10 $\mu$g, per plate, of the separately prepared DNA by the calcium phosphate procedure [see Haynes, J. & Weissmann, C., Nucleic Acids Res., 11, 687-706 (1983)]. After treatment with glycerol, the cells were incubated at 37° C for 12 hours.

The incubated cells were transferred onto three fresh plates (5 cm$^\varnothing$) and the media were changed twice a week. At day 16, the foci were transferred onto fresh plates and subjected to serial cultivation on a Dulbecco's minimal essential medium containing 10% bovine fetal serum (Gibco), so as to select clones having high G-CSF production rate. These clones produced G-CSF at a level of approximately 1 mg/L.

(B) Transformation of CHO cells and G-CSF (-VSE) expression therein

CHO cells (dhfr⁻ strain; courtesy of Dr. L. Chasin of Columbia University) were cultivated for growth in alpha-minimal essential medium containing 10% calf serum (a-MEN supplemented with adenosine. deoxyadenosine and thymidine) in plates (9 cm$^\varnothing$, Nunc). The cultured cells were transformed by the calcium phosphate procedure [Wigler et al., Cell, 14, 725 (1978)] in the following manner.

A carrier DNA (calf thymus DNA) was added in an appropriate amount to 1 μg of the plasmid pHGV2-dhfr prepared in Referential Example 10 (B), and the mixture was dissolved in 375 μl of a TE solution, followed by addition of 125 ul of 1 M CaCl$_2$. After the solution was cooled on ice for 3 - 5 minutes, 500 μl of 2 x HBS (50 mM Hepes, 280 mM NaCl, and 1.5 mM phosphate buffer) was added to the solution. After re-cooling on ice, the solution was mixed with 1 ml of the culture of CHO cells, transferred onto plates, and incubated for 9 hours in a CO$_2$ incubator. The medium was removed from the plate and, following washing with TBS (Tris-buffered saline), addition of 20% glycerol-containing TBS, and re-washing, a non-selective medium (the α-MEN medium described above except that it was supplemented with nucleotides) was added. After 2-day incubation, a 10-fold dilution of the culture was transferred onto a selective medium (not supplemented with nucleotides). The cultivation was continued, with the medium being replaced by a fresh selective medium every 2 days, and the resulting colonies were selected and transferred onto fresh plates, where the cells grew in the presence of 0.02 μM methotrexate (MTX), followed by cloning through growth in the presence of 0.05 μM MTX, which was later increased to 0.1 uM.

The transformation of CHO cells may also be accomplished by cotransformation with pHGV2 and pAdD26SVpA [see Scahill et al., Proc. Natl. Acad. Sci., USA, 80, 4654-4658 (1983)].

A recombinant vector that harbors a "polycistronic gene" may also be used to transform CHO cells. An example of this alternative method is as follows: pAdD26SVpA was treated with PstI and the recovered two fragments were joined to a pBRV2-derived CSF cDNA fragment so as to construct a recombinant vector wherein the adeno virus promoter, CSF cDNA, DHFR and the poly(A) site of SV40 were inserted in the order written. This recombinant vector was used to transform CHO cells.

Referential Example 12: Assay of G-CSF Activity (+ VSE and -VSE)

The supernatants of cultures of C127 cells and CHO cells which were obtained in Referential Examples 11 (A) and 11 (B), respectively, were adjusted to a pH of 4 with 1 N acetic acid. After addition of an equal volume of n-propanol, The resulting precipitate was removed by centrifugation The supernatant was passed through an open column (1$^\varnothing$ x 2 cm$^L$) fiiled with a C8 reverse-phased carrier (Yamamura Kagaku K.K.) and elution was conducted with 50% n-propanol. The eluate was diluted two-fold with water and subjected to reverse-phased high performance liquid chromatography on YMC-C8 column (Yamamura Kagaku K.K.), followed by elution with n-propanol (30 - 60% linear density gradient) containing 0.1% TFA. The fractions which were eluted at n-propanol concentrations of about 40% were recovered, freeze-dried and dissolved in 0.1 M glycine buffer (pH 9). As a result of these procedures, the human G-CSF in the C127 and CHO cells was concentrated about 20-fold.

As controls, cells were transformed with human G-CSF cDNA-free plasmids and the supernatants of their cultures were concentrated in accordance with the procedures described above. The human G-CSF activities of the samples were assayed by the method of human G-CSF activity assay (a) described earlier in this specification. If the efficiency of expression is adequately high, the supernatants of cultures may be directly assayed without being concentrated. The results are summarized in Table 2, wherein the data are based on concentrated samples.

Table 2

| Assay of Human G-CSF Activity | |
|---|---|
| | Human neutrophilic colonies (colonies/dish) |
| Purified human G-CSF (20 ng) | 96 |
| Culture of C127 cells transformed with pdBPV-1 (concentrated 20-fold) | 0 |

Table 2 (continued)

| Assay of Human G-CSF Activity | |
|---|---|
| | Human neutrophilic colonies (colonies/dish) |
| Culture of C127 cells transformed with pTNG4 (concentrated 20-fold) | 82 |
| Culture of CHO cells transformed with pAdD26SVpA (concentrated 20-fold) | 0 |
| Culture of CHO cells transformed with pHGV2-dhfr (concentrated 20-fold) | 111 |

Referential Example 13: Amino Acid Analysis and Sugar Analysis (+ VSE and -VSE)

1) Analysis of amino acid composition

The crude CSF samples prepared in Referential Example 12 were purified in accordance with the procedures described in Referential Example 2, (2), (iii). The purified CSF samples were subjected to analysis of amino acid composition by the same procedures and under the same conditions as those described in Referential Example 2, (2). (v). The results are shown in Table 3-(1) (+ VSE) and Table 3-(2) (-VSE).

Table 3-(1)

| Amino Acid Analysis Data (+ VSE) | |
|---|---|
| Amino acids | Mole% |
| Asp (Asp + Asn) | 2.3 |
| Thr | 3.9 |
| Ser | 8.5 |
| Glu (Glu + Gln) | 15.3 |
| Pro | 7.4 |
| Gly | 7.8 |
| Ala | 10.8 |
| 1/2 Cys | 2.8 |
| Val | 4.5 |
| Met | 1.7 |
| Ile | 2.3 |
| Leu | 18.6 |
| Tyr | 1.7 |
| Phe | 3.4 |
| Lys | 2.3 |
| His | 2.8 |
| Trp | 1.1 |
| Arg | 2.8 |

Table 3-(2)

| Amino Acid Analysis Data (-VSE) | |
|---|---|
| Amino acids | Mole% |
| Asp (Asp + Asn) | 2.3 |
| Thr | 4.0 |
| Ser | 8.1 |
| Glu (Glu + Gln) | 15.1 |
| Pro | 7.5 |

Table 3-(2)   (continued)

| Amino Acid Analysis Data (-VSE) | |
| --- | --- |
| Amino acids | Mole% |
| Gly | 8.0 |
| Ala | 10.9 |
| 1/2 Cys | 2.8 |
| Val | 3.9 |
| Met | 1.7 |
| Ile | 2.3 |
| Leu | 18.9 |
| Tyr | 1.7 |
| Phe | 3.5 |
| Lys | 2.3 |
| His | 2.9 |
| Trp | 1.2 |
| Arg | 2.9 |

2) Sugar composition analysis

An internal standard (25 nmol of inositol) was added to 200 ng of each of the purified CSF samples used in the analysis of amino acid composition 1). After addition of a methanol solution (500 μl) containing 1.5 N HCl, reaction was carried out at 90° C for 4 hours in a $N_2$ purged, closed tube. After the tube was opened. silver carbonate ($Ag_2CO_3$) was added to neutralize the contents. Thereafter, 50 μl of acetic anhydride was added and the tube was shaken for an adequate period. Subsequently, the tube was left overnight in the dark at room temperature. The upper layer was put into a sample tube and dried with a nitrogen gas. Methanol was added to the precipitate and the mixture was washed and lightly centrifuged. The upper layer was put into the same sample tube and dried. After addition of 50 μl of a TMS reagent (5:1:1 mixture of pyridine, hexamethyl disilazane and trimethylchlorosilane), reaction was carried out at 40°C for 20 minutes and the reaction product was stored in a deep freezer. A standard was prepared by combining 25 nmol of inositol with 50 nmol each of galactose (Gal), N-acetyl galactosamine (Gal NAc), sialic acid and any other appropriate reagents.

The samples thus prepared were subjected to gas chromatographic analysis under the following conditions:

Conditions of analysis

| | |
| --- | --- |
| Column | 2% OV - 17 VINport HP, 60 - 80 mesh, 3 m, glass |
| Temperature | elevated from 110 to 250°C at 4° C/min. |
| Carrier gas ($N_2$) pressure | initially 1.2 - 1.6 kg/cm$^2$<br>finally 2 - 2.5 kg/cm$^2$ |
| Sensitivity | $10^3$ MΩ range, 0.1 - 0.4 volts |
| Pressure | $H_2$, 0.8 kg/cm$^2$<br>air, 0.8 kg/cm$^2$ |
| Sample feed | 2.5 - 3.0 μl. |

As a result of the analysis, galactose, N-acetyl galactosamine and sialic acid, were identified in each of the CSF samples of the present invention.

3) Analysis of sugar content

Each of the CSF samples used in the analysis of sugar composition in 2) was subjected to quantitative analysis of amino sugar by the Elson-Morgan method, quantitative analysis of neutral sugar by the orcinol-sulfric acid method and quantitative analysis of sialic acid by the thiobarbituric acid method.

The procedures of the above analyses are described in Seikagaku Jikken Koza (Biochemical Experiment) Vol. 4, "Chemistry of Sugars (II)", Chap. 13 (Tokyo Kagaku Dojin).

Sugar content (weight %) of each of the samples was calculated on the basis of the measured data: the sugar content of the obtained G-CSF ranged from 1 to 20 wt% depending upon the host cell. the expression vector and the culture conditions.

Referential Example 14: Capability of Human G-CSF of the Present Invention to Increase the Number of Neutrophiles

Forty 8-week old male C57BL/6N mice were divided into two groups each consisting of 20 animals. One group (control group) was injected subcutaneously with 0.1 ml of a physiological saline solution containing n-propanol at a final concentration of 1% and C57BL/6N mouse serum at a final concentration of 10%. The other group (CSF treatment group) was injected subcutaneously with 0.1 ml of a physiological saline solution containing n-propanol at a final concentration of 1%, C57BL/6N mouse serum at a final concentration of 10% and 2.5 μg of the human G-CSF obtained in Referential Example 2. For each group. the injection was continued on a one-dose-a-day basis until blood sampling was conducted.

At day 2, 5, 8, 11 and 14 after the start of the injection, 4 mice were randomly sampled from each group and blood was taken from their supraorbital veins for counting the number of leukocytes with a microcell counter. Model CC180 (Toa K.K.) Blood smears were prepared and Giemsa staining was conducted in order to determine the proportion of neutrophiles in 200 leukocytes under the microscope. The number of neutrophiles in each group was calculated by the following formula:

$$\text{Neutrophile counts in 1 mm}^3 = \text{leukocyte counts in 1 mm}^3 \times \text{proportion of neutrophiles in leukocytes.}$$

A group of four C57BL/6N mice to which no injection was given at all were treated by the same procedures to obtain the values for day 0. The results are shown in Table 4.

The same test was conducted except that the human G-CSF was replaced by the pure samples for amino acid analysis that were used in Referential Example 13 (+VSE derived from C127 cells and -VSE derived from CHO cells). The results were identical to those shown in Table 4.

Table 4

| Days after injection | Control group (cells/mm$^3$) | CSF-treated group (cells/mm$^3$) |
|---|---|---|
| 0 | 765 ± 139 | 765 ± 139 |
| 2 | 1344 ± 389 | 3205 ± 439 |
| 5 | 1378 ± 474 | 4913 ± 530** |
| 8 | 1127 ± 242 | 3338 ± 310** |
| 11 | 965 ± 231 | 5229 ± 550*** |
| 14 | 1311 ± 197 | 5359 ± 584*** |

(Means ± standard error)
**P<0.01,
***P<0.001

Referential Example 15: Capability of Human G-CSF of the Present Invention to Inhibit Decrease in the Number of Leukocytes

Thirty-two 8-week old male ICR mice were injected peritoneally with 200 mg of cyclophosphamide (hereunder abbreviated as CP) per kg of mouse. The mice were then divided into two groups each consisting of 16 animals. One group (control group) was injected subcutaneously with 0.1 ml of a physiological saline solution containing n-propanol at a final concentration of 1% and IRC mouse serum at a final concentration of 10%. The other group (CSF treatment group) was injected subcutaneously with 0.1 ml of a physiological saline solution containing n-propanol at a final concentration of 1%, ICR mouse serum at a final concentration of 10%, and 2.5 μg of the human G-CSF obtained in Referential Example 2. For each group, the injection was continued on a one-dose-a-day basis until blood sampling was conducted.

At day 2, 4, 5 and 7 after the start of the CP injection, 4 mice were randomly sampled from each group and blood was taken from their supraorbital veins so that the number of leukocytes could be counted with a microcell counter, Model CC180 (Toa K.K.). Blood smears were prepared and Giemsa staining was conducted in order to determine the proportion of neutrophiles in 200 leukocytes under the microscope. The number of neutrophiles in each group was calculated by the following formula:

$$\text{Neutrophile counts in 1 mm}^3 = \text{leukocyte counts in 1 mm}^3 \text{ x proportion of neutrophiles in leukocytes.}$$

The stained neutrophiles were classified morphologically in order to determine the proportion of segmented neutrophiles which <u>were the most mature form of neutrophiles</u>.

A group of four C57BL/6N mice to which no injection was given at all were treated by the same procedures to obtain the values for day 0. The results are shown in Table 5. The same test was conducted employing the pure samples of human G-CSF used in amino acid analysis in Referential Example 13 (+VSE derived from C127 cells and -VSE from CHO cells). The results were identical to those shown in Table 5.

Table 5

| Control group | | | |
|---|---|---|---|
| Days | leukocyte count (cell/mm$^3$) | neutrophile count (cell/mm$^3$) | proportion of segmented neutrophiles (%) |
| 0 | $7800 \pm 297$ | $1068 \pm 131$ | $84 \pm 4$ |
| 2 | $2200 \pm 187$ | $866 \pm 131$ | $91 \pm 3$ |
| 4 | $1650 \pm 132$ | $59 \pm 4$ | $29 \pm 14$ |
| 5 | $2050 \pm 253$ | $236 \pm 36$ | $28 \pm 4$ |
| 7 | $3775 \pm 551$ | $1042 \pm 462$ | $38 \pm 5$ |
| CSF treatment group | | | |
| Days | leukocyte count (cell/mm$^3$) | neutrophile count (cell/mm$^3$) | proportion of segmented neutrophiles (%) |
| 0 | $7800 \pm 297$ | $1068 \pm 131$ | $84 \pm 4$ |
| 2 | $3750 \pm 724$ | $2417 \pm 708$ | $98 \pm 1$ |
| 4 | $3975 \pm 103$*** | $1492 \pm 129$*** | $54 \pm 6$*** |
| 5 | $7100 \pm 1127$** | $5017 \pm 959$** | $89 \pm 7$** |
| 7 | $3725 \pm 347$ | $965 \pm 175$ | $58 \pm 5$ |

(Mean ± standard error) **P<0.01,
***P<0.001

As Table 4 shows, no increase in the neutrophile count was observed in the control group of normal animals but, in the CSF-treated group, a significant increase in the neutrophile count was observed at day 2 after the administration of CSF and a gradual increase occurred thereafter so long as the CSF supply was continued. As is clear from Table 5 showing the results for animal models with neutropenia, the decrease in the neutrophile count was continued in the control group until day 5 after the CP administration but, in the CSF treated group, the neutrophile counts observed for period of 7 days after the CP administration were equal to or higher than the initial values.

The improvement achieved by the administration of human G-CSF was apparent not only for the absolute number of peripheral neutrophiles but also with respect to their maturity. The decrease in the number of peripheral leukocytes was also mitigated by the administration of human G-CSF.

<u>Referential Example 16</u>: Effect of human G-CSF on leukopenic models

Thirty-six C57BL/6N mice (male, 8-week old) were injected intraperitoneally with 200 mg/kg of CP. Four days later, 4 mice were randomly sampled and blood was taken from their supraorbital veins so that it could be verified that the numbers of peripheral leukocytes and neutrophiles had been adequately lowered. The remaining 32 mice were divided into two groups each consisting of 16 animals. Starting on the next day, one group (control group) was injected subcutaneously with 0.1 ml of a physiological saline solution containing n-propanol at a final concentration of 1% and C57BL/6N mouse serum at a final concentration of 10%, and the other group (CSF treatment group) was injected subcutaneously with 0.1 ml of a physiological saline solution containing n-propanol at a final concentration of 1%, C57BL/6N mouse serum at a final concentration of 10%, and 2.5 μg of the human G-CSF obtained in Referential Example 2. For each group, the injection was continued for 2 days on a one-dose-a-day basis. On predetermined days, 4 mice were randomly sampled from each group and blood was taken from their supraorbital veins so that the numbers of leukocytes and neutrophiles could be determined. In this experiment. all administrations were conducted at 9 o'clock a.m. and all blood samples were taken at 3 p.m. Counting of the leukocytes in the blood samples, their classification and the determination of the number of peripheral neutrophiles were conducted as in Referential Example 15. A group of four C57BL/6N mice to which no injection was given at all were treated by the same proceures to obtain the values for day 0. In this experiment, only one blood sample was taken from each mouse.

The results of the experiment are shown in Table 6. The same experiment was repeated using the pure samples of human G-CSF that were employed for amino acid assaying in Referential Example 13 (+VSE derived from C127 cells and -VSE from CHO cells). The results obtained were identical to those shown in Table 6.

Table 6

| Peripheral leukocyte count (cell/mm$^3$) | | |
|---|---|---|
| Days | control group | CSF treated group |
| 0 | 9864 ± 1121 | |
| 4 | 1577 ± 266 | |
| 5 | 3061 ± 374 | 7946 ± 504*** |
| 6 | 4866 ± 701 | 29113 ± 1544*** |
| 7 | 8212 ± 1824 | 8300 ± 3210 |
| 11 | 10207 ± 984 | 11544 ± 844 |
| Peripheral neutrophile count (cell/mm$^3$) | | |
| Days | control group | CSF treated group |
| 0 | 1162 ± 104 | |
| 4 | 68 ± 7 | |
| 5 | 236 ± 36 | 1208 ± 25*** |
| 6 | 483 ± 42 | 24168 ± 173*** |
| 7 | 3062 ± 462 | 5746 ± 1920 |
| 11 | 987 ± 49 | 1055 ± 105 |

(Mean ± standard error) ***P<0.001

As Table 6 shows, the administration of G-CSF induced a distinct recovery from the decrease in the numbers of leukocytes and neutrophiles.

In this connection, it should be emphasized that the aforementioned results were attained not only with the human G-CSF obtained from the supernatant of the culture of human G-CSF producing cells but also with the human G-CSF that the assignee of the present invention successfully produced from the transformants harboring the gene coding for said human G-CSF.

These results suggest the effectiveness of the human G-CSF of the present invention as a neutropenia treating agent.

It should also be mentioned that no toxicity was found under the conditions employed in the experiments described above.

Example 1

The human G-CSF containing fraction obtained by the method described in Referential Example 2 was rendered germ-free and frozen at -20° C. The frozen fraction was worked up to prepare an injection.

Example 2

The C127 cell-derived human G-CSF of +VSE line obtained in Referential Example 12 was purified by the method described in Referential Example 2, (2), (iii), aseptically charged in amounts of 5 ml in 10-ml vials, and freeze-dried at -20° C, with the vials being subsequently closed with rubber stoppers. The so obtained freeze-dried products were worked up to prepare an injection preparation.

Example 3

The CHO cell-derived human G-CSF of -VSE line obtained in Referential Example 12 was purified by the method described in Referential Example 2, (2), (iii), aseptically charged in amounts of 5 ml in 10-ml vials and freeze-dried at -20° C, with the vials being subsequently closed with rubber stoppers. The so obtained freeze-dried products were worked up to prepare an injection preparation.

## Claims

**Claims for the following Contracting States : BE, CH, DE, FR, GB, IT, LI, NL, SE**

1. Use of a human G-CSF for the preparation of a pharmaceutical composition for the treatment of neutropenia by increasing the number of neutrophiles having a fully matured form.

2. Use according to claim 1 wherein the human G-CSF is present in an amount of 0.01-500 μg.

3. Use according to claim 1 and 2 wherein the human G-CSF is present in an amount of 0.5 to 200 μg.

4. Use according to claim 1 wherein the human granulocyte colony stimulating factor is obtained from the supernatant of the culture of a human granulocyte colony stimulating factor producing cell.

5. Use according to claim 4 wherein the human granulocyte colony stimulating factor has the following physicochemical properties:

   i) molecular weight: about $19.000 \pm 1,000$ as measured by electrophoresis through a sodium dodecylsulfate - polyacrylamide gel;
   ii) isoelectric point: having at least one of the three isoelectric points, pI $= 5.5 \pm 0.1$, pI $= 5.8 \pm 0.1$, and pI $= 6.1 \pm 0.1$;
   iii) ultraviolet absorption: having a maximum absorption at 280 nm and a minimum absorption at 250 nm;
   iv) amino acid sequence of the 21 residues from N terminus:

$$H_2N - Thr - Pro - Leu - Gly - Pro - Ala - Ser - Ser -$$
$$Leu - Pro - Gln - Ser - Phe - Leu - Leu - Lys - Cys -$$
$$Leu - Glu - Gln - Val$$

6. Use according to claim 1 wherein the human granulocyte colony stimulating factor is a polypeptide or glycoprotein having the human granulocyte colony stimulating factor activity which is produced from a transformant containing a recombinant vector harboring a gene coding for the polypeptide having the human granulocyte colony stimulating factor activity.

7. Use according to claim 6 wherein the gene coding for the polypeptide having the human granulocyte colony stimulating factor activity has all or part of the nucleotide sequence shown in accompanying Fig. 2.

8. Use according to claim 6 wherein the gene coding for the polypeptide having the human granulocyte colony stimulating factor activity has all or part of the nucleotide sequence shown in accompanying Fig. 3.

9. Use according to claim 6 wherein the polypeptide having the human granulocyte colony stimulating factor activity is represented by all or part of the amino acid sequence shown below:

$$(Met)_n Thr \quad Pro \quad Leu \quad Gly \quad Pro \quad Ala \quad Ser \quad Ser \quad Leu \quad Pro$$
$$Gln \quad Ser \quad Phe \quad Leu \quad Leu \quad Lys \quad Cys \quad Leu \quad Glu \quad Gln \quad Val$$
$$Arg \quad Lys \quad Ile \quad Gln \quad Gly \quad Asp \quad Gly \quad Ala \quad Ala \quad Leu \quad Gln$$
$$Glu \quad Lys \quad Leu \quad (Val \quad Ser \quad Glu)_m Cys \quad Ala \quad Thr \quad Tyr \quad Lys$$
$$Leu \quad Cys \quad His \quad Pro \quad Glu \quad Glu \quad Leu \quad Val \quad Leu \quad Leu \quad Gly$$

```
His   Ser   Leu   Gly   Ile   Pro   Trp   Ala   Pro   Leu   Ser

Ser   Cys   Pro   Ser   Gln   Ala   Leu   Gln   Leu   Ala   Gly

Cys   Leu   Ser   Gln   Leu   His   Ser   Gly   Leu   Phe   Leu

Tyr   Gln   Gly   Leu   Leu   Gln   Ala   Leu   Glu   Gly   Ile

Ser   Pro   Glu   Leu   Gly   Pro   Thr   Leu   Asp   Thr   Leu

Gln   Leu   Asp   Val   Ala   Asp   Phe   Ala   Thr   Thr   Ile

Trp   Gln   Gln   Met   Glu   Glu   Leu   Gly   Met   Ala   Pro

Ala   Leu   Gln   Pro   Thr   Gln   Gly   Ala   Met   Pro   Ala

Phe   Ala   Ser   Ala   Phe   Gln   Arg   Arg   Ala   Gly   Gly

Val   Leu   Val   Ala   Ser   His   Leu   Gln   Ser   Phe   Leu

Glu   Val   Ser   Tyr   Arg   Val   Leu   Arg   His   Leu   Ala

Gln   Pro
```

(provided that m is 0 or 1; and n is 0 or 1).

10. Use according to Claim 6 wherein the glycoprotein having the human granulocyte colony stimulating factor activity has a polypeptide and a sugar chain portion, said polypeptide being represented by all or part of the amino acid sequence shown below:

```
Thr   Pro   Leu   Gly   Pro   Ala   Ser   Ser   Leu   Pro   Gln

Ser   Phe   Leu   Leu   Lys   Cys   Leu   Glu   Gln   Val   Arg

Lys   Ile   Gln   Gly   Asp   Gly   Ala   Ala   Leu   Gln   Glu

Lys   Leu   (Val   Ser   Glu)  Cys   Ala   Thr   Tyr   Lys   Leu
                            m
Cys   His   Pro   Glu   Glu   Leu   Val   Leu   Leu   Gly   His


Ser   Leu   Gly   Ile   Pro   Trp   Ala   Pro   Leu   Ser   Ser

Cys   Pro   Ser   Gln   Ala   Leu   Gln   Leu   Ala   Gly   Cys

Leu   Ser   Gln   Leu   His   Ser   Gly   Leu   Phe   Leu   Tyr

Gln   Gly   Leu   Leu   Gln   Ala   Leu   Glu   Gly   Ile   Ser

Pro   Glu   Leu   Gly   Pro   Thr   Leu   Asp   Thr   Leu   Gln

Leu   Asp   Val   Ala   Asp   Phe   Ala   Thr   Thr   Ile   Trp

Gln   Gln   Met   Glu   Glu   Leu   Gly   Met   Ala   Pro   Ala

Leu   Gln   Pro   Thr   Gln   Gly   Ala   Met   Pro   Ala   Phe
```

```
Ala   Ser   Ala   Phe   Gln   Arg   Arg   Ala   Gly   Gly   Val
Leu   Val   Ala   Ser   His   Leu   Gln   Ser   Phe   Leu   Glu
Val   Ser   Tyr   Arg   Val   Leu   Arg   His   Leu   Ala   Gln
Pro
```

(provided that m is 0 or 1).

**Claims for the following Contracting States : AT, ES**

1. A method for the preparation of a pharmaceutical composition for the treatment of neutropenia by increasing the number of neutrophiles having a fully matured form, comprising combining human G-CSF with a pharmaceutically acceptable carrier or excipient and optionally a stabilizer and/or an anti-adsorption agent.

2. The Method according to claim 1 wherein the human G-CSF is present in an amount of 0.01-500 µg.

3. The Method according to claim 1 and 2 wherein the human G-CSF is present in an amount of 0.5 to 200 µg.

4. The Method according to claim 1 wherein the human granulocyte colony stimulating factor is obtained from the supernatant of the culture of a human granulocyte colony stimulating factor producing cell.

5. The Method according to claim 4 wherein the human granulocyte colony stimulating factor has the following physicochemical properties:

    i) molecular weight: about $19.000 \pm 1,000$ as measured by electrophoresis through a sodium dodecylsulfate - polyacrylamide gel;
    ii) isoelectric point: having at least one of the three isoelectric points, $pI = 5.5 \pm 0.1$, $pI = 5.8 \pm 0.1$, and $pI = 6.1 \pm 0.1$;
    iii) ultraviolet absorption: having a maximum absorption at 280 nm and a minimum absorption at 250 nm;
    iv) amino acid sequence of the 21 residues from N terminus:

```
H_2N - Thr - Pro - Leu - Gly - Pro - Ala - Ser - Ser -
Leu - Pro - Gln - Ser - Phe - Leu - Leu - Lys - Cys -
Leu - Glu - Gln - Val
```

6. The Method according to claim 1 wherein the human granulocyte colony stimulating factor is a polypeptide or glycoprotein having the human granulocyte colony stimulating factor activity which is produced from a transformant containing a recombinant vector harboring a gene coding for the polypeptide having the human granulocyte colony stimulating factor activity.

7. The Method according to claim 6 wherein the gene coding for the polypeptide having the human granulocyte colony stimulating factor activity has all or part of the nucleotide sequence shown in accompanying Fig. 2.

8. The Method according to claim 6 wherein the gene coding for the polypeptide having the human granulocyte colony stimulating factor activity has all or part of the nucleotide sequence shown in accompanying Fig. 3.

9. The Method according to claim 6 wherein the polypeptide having the human granulocyte colony stimulating factor activity is represented by all or part of the amino acid sequence shown below:

```
(Met)_n Thr  Pro  Leu  Gly  Pro  Ala  Ser  Ser  Leu  Pro
        Gln  Ser  Phe  Leu  Leu  Lys  Cys  Leu  Glu  Gln  Val
        Arg  Lys  Ile  Gln  Gly  Asp  Gly  Ala  Ala  Leu  Gln
        Glu  Lys  Leu  (Val Ser  Glu)_m Cys Ala  Thr  Tyr  Lys
        Leu  Cys  His  Pro  Glu  Glu  Leu  Val  Leu  Leu  Gly


        His  Ser  Leu  Gly  Ile  Pro  Trp  Ala  Pro  Leu  Ser
        Ser  Cys  Pro  Ser  Gln  Ala  Leu  Gln  Leu  Ala  Gly
        Cys  Leu  Ser  Gln  Leu  His  Ser  Gly  Leu  Phe  Leu
        Tyr  Gln  Gly  Leu  Leu  Gln  Ala  Leu  Glu  Gly  Ile
        Ser  Pro  Glu  Leu  Gly  Pro  Thr  Leu  Asp  Thr  Leu
        Gln  Leu  Asp  Val  Ala  Asp  Phe  Ala  Thr  Thr  Ile
        Trp  Gln  Gln  Met  Glu  Glu  Leu  Gly  Met  Ala  Pro
        Ala  Leu  Gln  Pro  Thr  Gln  Gly  Ala  Met  Pro  Ala
        Phe  Ala  Ser  Ala  Phe  Gln  Arg  Arg  Ala  Gly  Gly
        Val  Leu  Val  Ala  Ser  His  Leu  Gln  Ser  Phe  Leu
        Glu  Val  Ser  Tyr  Arg  Val  Leu  Arg  His  Leu  Ala
        Gln  Pro
```

(provided that m is 0 or 1; and n is 0 or 1).

10. The Method according to Claim 6 wherein the glycoprotein having the human granulocyte colony stimulating factor activity has a polypeptide and a sugar chain portion, said polypeptide being represented by all or part of the amino acid sequence shown below:

```
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  (Val  Ser  Glu)  Cys  Ala  Thr  Tyr  Lys  Leu
                        m
Cys  His  Pro  Glu  Glu  Leu  Val  Leu  Leu  Gly  His

Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe


Ala  Ser  Ala  Phe  Gln  Arg  Arg  Ala  Gly  Gly  Val
Leu  Val  Ala  Ser  His  Leu  Gln  Ser  Phe  Leu  Glu
Val  Ser  Tyr  Arg  Val  Leu  Arg  His  Leu  Ala  Gln
Pro
```

(provided that m is 0 or 1).

**Patentansprüche**

**Patentansprüche für folgende Vertragsstaaten : BE, CH, DE, FR, GB, IT, LI, NL, SE**

1.  Verwendung eines menschlichen G-CSF für die Herstellung eines Arzneimittels zur Behandlung von Neutropenie durch Erhöhung der Anzahl von Neutrophilen, die voll ausgereift sind.

2.  Verwendung nach Anspruch 1, wobei der menschliche G-CSF in einer Menge von 0,01 bis 500 µg anwesend ist.

3.  Verwendung nach Anspruch 1 und 2, wobei der menschliche G-CSF in einer Menge von 0,5 bis 200 µg anwesend ist.

4.  Verwendung nach Anspruch 1, wobei der menschliche Granulocyten-Kolonie-stimulierende Faktor vom Kulturüberstand einer menschlichen Granulocyten-Kolonie-stimulierenden Faktor herstellenden Zelle erhalten wird.

5.  Verwendung nach Anspruch 4, wobei der menschliche Granulocyten-Kolonie-stimulierende Faktor die nachfolgenden physikalisch-chemischen Eigenschaften aufweist:

    i) Molekulargewicht: etwa 19 000 ± 1000, nach Bestimmung durch Elektrophorese in einem Natriumdodecylsulfat-Polyacrylamidgel;
    ii) Isoelektrischer Punkt: er weist mindestens einen der drei isoelektrische Punkte pI = 5,5 ± 0,1, pI = 5,8 ±

0,1 und pl = 6,1 ± 0,1 auf;

iii) Ultraviolette Absorption: er weist eine maximale Absorption bei 280 nm und eine minimale Absorption bei 250 nm auf;

iv) Aminosäuresequenz der 21 N-terminalen Reste:

$$H_2N - Thr - Pro - Leu - Gly - Pro - Ala - Ser - Ser -$$

$$Leu - Pro - Gln - Ser - Phe - Leu - Leu - Lys - Cys -$$

$$Leu - Glu - Gln - Val.$$

**6.** Verwendung nach Anspruch 1, wobei der menschliche Granulocyten-Kolonie-stimulierende Faktor ein Polypeptid oder Glycoprotein mit der Aktivität des menschlichen Granulocyten-Kolonie-stimulierenden Faktors ist, das von einer Transformante hergestellt wird, die einen rekombinanten Vektor enthält, der ein ein Polypeptid mit der Aktivität des menschlichen Granulocyten-Kolonie-stimulierenden Faktors codierendes Gen trägt.

**7.** Verwendung nach Anspruch 6, wobei das das Polypeptid mit der Aktivität des menschlichen Granulocyten-Kolonie-stimulierenden Faktors codierende Gen die gesamte oder einen Teil der in der beigefügten Figur 2 dargestellten Nucleotidsequenz aufweist.

**8.** Verwendung nach Anspruch 6, wobei das das Polypeptid mit der Aktivität des menschlichen Granulocyten-Kolonie-stimulierenden Faktors codierende Gen die gesamte oder einen Teil der in der beigefügten Figur 3 dargestellten Nucleotidsequenz aufweist.

**9.** Verwendung nach Anspruch 6, wobei das Polypeptid mit der Aktivität des menschlichen Granulocyten-Kolonie-stimulierenden Faktors durch die gesamte oder einen Teil der nachfolgend dargestellten Aminosäuresequenz verkörpert wird:

```
(Met)_n Thr   Pro   Leu   Gly   Pro   Ala   Ser   Ser   Leu   Pro
        Gln   Ser   Phe   Leu   Leu   Lys   Cys   Leu   Glu   Gln   Val
        Arg   Lys   Ile   Gln   Gly   Asp   Gly   Ala   Ala   Leu   Gln
        Glu   Lys   Leu   (Val   Ser   Glu)_m Cys   Ala   Thr   Tyr   Lys
        Leu   Cys   His   Pro   Glu   Glu   Leu   Val   Leu   Leu   Gly
        His   Ser   Leu   Gly   Ile   Pro   Trp   Ala   Pro   Leu   Ser
```

| | | | | | | | | | |
|---|---|---|---|---|---|---|---|---|---|
| Ser | Cys | Pro | Ser | Gln | Ala | Leu | Gln | Leu | Ala | Gly |
| Cys | Leu | Ser | Gln | Leu | His | Ser | Gly | Leu | Phe | Leu |
| Tyr | Gln | Gly | Leu | Leu | Gln | Ala | Leu | Glu | Gly | Ile |
| Ser | Pro | Glu | Leu | Gly | Pro | Thr | Leu | Asp | Thr | Leu |
| Gln | Leu | Asp | Val | Ala | Asp | Phe | Ala | Thr | Thr | Ile |
| Trp | Gln | Gln | Met | Glu | Glu | Leu | Gly | Met | Ala | Pro |
| Ala | Leu | Gln | Pro | Thr | Gln | Gly | Ala | Met | Pro | Ala |
| Phe | Ala | Ser | Ala | Phe | Gln | Arg | Arg | Ala | Gly | Gly |
| Val | Leu | Val | Ala | Ser | His | Leu | Gln | Ser | Phe | Leu |
| Glu | Val | Ser | Tyr | Arg | Val | Leu | Arg | His | Leu | Ala |
| Gln | Pro | | | | | | | | | |

(vorausgesetzt, daß m 0 oder 1 ist und n 0 oder 1 ist).

10. Verwendung nach Anspruch 6, wobei das Glycoprotein mit der Aktivität des menschlichen Granulocyten-Kolonie-stimulierenden Faktors einen Polypeptid- und einen Zuckerkettenanteil aufweist, wobei das Polypeptid durch die gesamte oder einen Teil der nachfolgend dargestellten Aminosäuresequenz verkörpert wird:

| | | | | | | | | | |
|---|---|---|---|---|---|---|---|---|---|
| Thr | Pro | Leu | Gly | Pro | Ala | Ser | Ser | Leu | Pro | Gln |
| Ser | Phe | Leu | Leu | Lys | Cys | Leu | Glu | Gln | Val | Arg |
| Lys | Ile | Gln | Gly | Asp | Gly | Ala | Ala | Leu | Gln | Glu |
| Lys | Leu | (Val | Ser | Glu)$_m$ | Cys | Ala | Thr | Tyr | Lys | Leu |
| Cys | His | Pro | Glu | Glu | Leu | Val | Leu | Leu | Gly | His |
| Ser | Leu | Gly | Ile | Pro | Trp | Ala | Pro | Leu | Ser | Ser |
| Cys | Pro | Ser | Gln | Ala | Leu | Gln | Leu | Ala | Gly | Cys |
| Leu | Ser | Gln | Leu | His | Ser | Gly | Leu | Phe | Leu | Tyr |
| Gln | Gly | Leu | Leu | Gln | Ala | Leu | Glu | Gly | Ile | Ser |
| Pro | Glu | Leu | Gly | Pro | Thr | Leu | Asp | Thr | Leu | Gln |
| Leu | Asp | Val | Ala | Asp | Phe | Ala | Thr | Thr | Ile | Trp |
| Gln | Gln | Met | Glu | Glu | Leu | Gly | Met | Ala | Pro | Ala |

| | | | | | | | | | |
|---|---|---|---|---|---|---|---|---|---|
| Leu | Gln | Pro | Thr | Gln | Gly | Ala | Met | Pro | Ala | Phe |
| Ala | Ser | Ala | Phe | Gln | Arg | Arg | Ala | Gly | Gly | Val |
| Leu | Val | Ala | Ser | His | Leu | Gln | Ser | Phe | Leu | Glu |
| Val | Ser | Tyr | Arg | Val | Leu | Arg | His | Leu | Ala | Gln |
| Pro | | | | | | | | | | |

(vorausgesetzt, daß m 0 oder 1 ist).

**Patentansprüche für folgende Vertragsstaaten : AT, ES**

1. Verfahren zur Herstellung eines Arzneimittels zur Behandlung der Neutropenie durch Erhöhung der Anzahl von Neutrophilen, die völlig ausgereift sind, umfassend das Kombinieren von menschlichem G-CSF mit einem pharmazeutisch verträglichen Träger oder Exzipienten und, gegebenenfalls, einem Stabilisator und/oder einem Anti-Adsorptionswirkstoff.

2. Verfahren nach Anspruch 1, wobei der menschliche G-CSF in einer Menge von 0,01 bis 500 μg anwesend ist.

3. Verfahren nach Anspruch 1 und 2, wobei der menschliche G-CSF in einer Menge von 0,5 bis 200 μg anwesend ist.

4. Verfahren nach Anspruch 1, wobei der menschliche Granulocyten-Kolonie-stimulierende Faktor vom Kulturüberstand einer menschlichen Granulocyten-Kolonie-stimulierenden Faktor herstellenden Zelle erhalten wird.

5. Verfahren nach Anspruch 4, wobei der menschliche Granulocyten-Kolonie-stimulierende Faktor die nachfolgenden physikalisch-chemischen Eigenschaften aufweist:

    i) Molekulargewicht: etwa 19 000 ± 1000, nach Bestimmung durch Elektrophorese in einem Natriumdodecylsulfat-Polyacrylamidgel;
    ii) Isoelektrischer Punkt: er weist mindestens einen der drei isoelektrische Punkte pI = 5,5 ± 0,1, pI = 5,8 ± 0,1 und pI = 6,1 ± 0,1 auf;
    iii) Ultraviolette Absorption: er weist eine maximale Absorption bei 280 nm und eine minimale Absorption bei 250 nm auf;
    iv) Aminosäuresequenz der 21 N-terminalen Reste:

```
H2N - Thr - Pro - Leu - Gly - Pro - Ala - Ser - Ser -

Leu - Pro - Gln - Ser - Phe - Leu - Leu - Lys - Cys -

Leu - Glu - Gln - Val.
```

6. Verfahren nach Anspruch 1, wobei der menschliche Granulocyten-Kolonie-stimulierende Faktor ein Polypeptid oder Glycoprotein mit der Aktivität des menschlichen Granulocyten-Kolonie-stimulierenden Faktors ist, das von einer Transformante hergestellt wird, die einen rekombinanten Vektor enthält, der ein ein Polypeptid mit der Aktivität des menschlichen Granulocyten-Kolonie-stimulierenden Faktors codierendes Gen trägt.

7. Verfahren nach Anspruch 6, wobei das das Polypeptid mit der Aktivität des menschlichen Granulocyten-Kolonie-stimulierenden Faktors codierende Gen die gesamte oder einen Teil der in der beigefügten Figur 2 dargestellten Nucleotidsequenz aufweist.

8. Verfahren nach Anspruch 6, wobei das das Polypeptid mit der Aktivität des menschlichen Granulocyten-Kolonie-stimulierenden Faktors codierende Gen die gesamte oder einen Teil der in der beigefügten Figur 3 dargestellten Nucleotidsequenz aufweist.

9. Verfahren nach Anspruch 6, wobei das Polypeptid mit der Aktivität des menschlichen Granulocyten-Kolonie-stimulierenden Faktors durch die gesamte oder einen Teil der nachfolgend dargestellten Aminosäuresequenz verkörpert wird:

| (Met)$_n$Thr | Pro | Leu | Gly | Pro | Ala | Ser | Ser | Leu | Pro |
|---|---|---|---|---|---|---|---|---|---|
| Gln | Ser | Phe | Leu | Leu | Lys | Cys | Leu | Glu | Gln | Val |
| Arg | Lys | Ile | Gln | Gly | Asp | Gly | Ala | Ala | Leu | Gln |
| Glu | Lys | Leu | (Val | Ser | Glu)$_m$ | Cys | Ala | Thr | Tyr | Lys |
| Leu | Cys | His | Pro | Glu | Glu | Leu | Val | Leu | Leu | Gly |
| His | Ser | Leu | Gly | Ile | Pro | Trp | Ala | Pro | Leu | Ser |
| Ser | Cys | Pro | Ser | Gln | Ala | Leu | Gln | Leu | Ala | Gly |
| Cys | Leu | Ser | Gln | Leu | His | Ser | Gly | Leu | Phe | Leu |
| Tyr | Gln | Gly | Leu | Leu | Gln | Ala | Leu | Glu | Gly | Ile |
| Ser | Pro | Glu | Leu | Gly | Pro | Thr | Leu | Asp | Thr | Leu |
| Gln | Leu | Asp | Val | Ala | Asp | Phe | Ala | Thr | Thr | Ile |
| Trp | Gln | Gln | Met | Glu | Glu | Leu | Gly | Met | Ala | Pro |
| Ala | Leu | Gln | Pro | Thr | Gln | Gly | Ala | Met | Pro | Ala |
| Phe | Ala | Ser | Ala | Phe | Gln | Arg | Arg | Ala | Gly | Gly |
| Val | Leu | Val | Ala | Ser | His | Leu | Gln | Ser | Phe | Leu |
| Glu | Val | Ser | Tyr | Arg | Val | Leu | Arg | His | Leu | Ala |
| Gln | Pro | | | | | | | | | |

(vorausgesetzt, daß m 0 oder 1 ist und n 0 oder 1 ist).

**10.** Verfahren nach Anspruch 6, wobei das Glycoprotein mit der Aktivität des menschlichen Granulocyten-Kolonie-stimulierenden Faktors einen Polypeptid- und einen Zuckerkettenanteil aufweist, wobei das Polypeptid durch die gesamte oder einen Teil der nachfolgend dargestellten Aminosäuresequenz verkörpert wird:

| Thr | Pro | Leu | Gly | Pro | Ala | Ser | Ser | Leu | Pro | Gln |
|---|---|---|---|---|---|---|---|---|---|---|
| Ser | Phe | Leu | Leu | Lys | Cys | Leu | Glu | Gln | Val | Arg |
| Lys | Ile | Gln | Gly | Asp | Gly | Ala | Ala | Leu | Gln | Glu |
| Lys | Leu | (Val | Ser | Glu)$_m$ | Cys | Ala | Thr | Tyr | Lys | Leu |
| Cys | His | Pro | Glu | Glu | Leu | Val | Leu | Leu | Gly | His |
| Ser | Leu | Gly | Ile | Pro | Trp | Ala | Pro | Leu | Ser | Ser |
| Cys | Pro | Ser | Gln | Ala | Leu | Gln | Leu | Ala | Gly | Cys |
| Leu | Ser | Gln | Leu | His | Ser | Gly | Leu | Phe | Leu | Tyr |

| Gln | Gly | Leu | Leu | Gln | Ala | Leu | Glu | Gly | Ile | Ser |
| Pro | Glu | Leu | Gly | Pro | Thr | Leu | Asp | Thr | Leu | Gln |
| Leu | Asp | Val | Ala | Asp | Phe | Ala | Thr | Thr | Ile | Trp |
| Gln | Gln | Met | Glu | Glu | Leu | Gly | Met | Ala | Pro | Ala |
| Leu | Gln | Pro | Thr | Gln | Gly | Ala | Met | Pro | Ala | Phe |
| Ala | Ser | Ala | Phe | Gln | Arg | Arg | Ala | Gly | Gly | Val |
| Leu | Val | Ala | Ser | His | Leu | Gln | Ser | Phe | Leu | Glu |
| Val | Ser | Tyr | Arg | Val | Leu | Arg | His | Leu | Ala | Gln |
| Pro |

(vorausgesetzt, daß m 0 oder 1 ist).

**Revendications**

**Revendications pour les Etats contractants suivants : BE, CH, DE, FR, GB, IT, LI, NL, SE**

1. Utilisation d'un facteur de stimulation de colonie de granulocytes humains pour la préparation d'une composition pharmaceutique pour le traitement de la neutropénie par augmentation du nombre de neutrophiles ayant une forme à complète maturité.

2. Utilisation suivant la revendication 1, dans laquelle le facteur de stimulation de colonie de granulocytes humains est présent en une quantité de 0,01 à 500 µg.

3. Utilisation suivant les revendications 1 et 2, dans laquelle le facteur de stimulation de colonie de granulocytes humains est présent en une quantité de 0,5 à 200 µg.

4. Utilisation suivant la revendication 1, dans laquelle le facteur de stimulation de colonie de granulocytes humains est obtenu à partir du surnageant de la culture d'une cellule produisant le facteur de stimulation de colonie de granulocytes humains.

5. Utilisation suivant la revendication 4, dans laquelle le facteur de stimulation de colonie de granulocytes humains a les propriétés physico-chimiques suivantes :

    i) poids moléculaire
    environ 19 000 ± 1 000 tel que mesuré par électrophorèse dans un gel de polyacrylamide avec dodécylsulfate de sodium;
    ii) point isoélectrique :
    ayant au moins l'un des trois points isoélectriques, pl = 5,5 ± 0,1, pl = 5,8 ± 0,1 et pl = 6,1 1 0,1;
    iii) absorption dans l'ultraviolet :
    ayant un maximum d'absorption à 280 nm et un minimum d'absorption à 250 nm;
    iv) séquence d'acides aminés des 21 résidus depuis l'extrémité N-terminale :

```
H₂N - Thr - Pro - Leu - Gly - Pro - Ala - Ser - Ser -
Leu - Pro - Gln - Ser - Phe - Leu - Leu - Lys - Cys -
Leu - Glu - Gln - Val.
```

6. Utilisation suivant la revendication 1, dans laquelle le facteur de stimulation de colonie de granulocytes humains est un polypeptide ou une glycoprotéine ayant l'activité de facteur de stimulation de colonie de granulocytes humains qui est produit par un transformant contenant un vecteur recombinant hébergeant un gène codant pour le polypeptide ayant l'activité de facteur de stimulation de colonie de granulocytes humains.

7. Utilisation suivant la revendication 6, dans laquelle le gène codant pour le polypeptide ayant l'activité de facteur de stimulation de colonie de granulocytes humains a tout ou partie de la séquence de nucléotides représentée à la Fig. 2 annexée.

8. Utilisation suivant la revendication 6, dans laquelle le gène codant pour le polypeptide ayant l'activité de facteur de stimulation de colonie de granulocytes humains a tout ou partie de la séquence de nucléotides représentée à la Fig. 3 annexée.

9. Utilisation suivant la revendication 6, dans laquelle le polypeptide ayant l'activité de facteur de stimulation de colonie de granulocytes humains est représenté par tout ou partie de la séquence d'acides aminés indiquée ci-après :

```
(Met)ₙThr  Pro  Leu  Gly  Pro  Ala  Ser  Ser  Leu  Pro
     Gln  Ser  Phe  Leu  Leu  Lys  Cys  Leu  Glu  Gln  Val
     Arg  Lys  Ile  Gln  Gly  Asp  Gly  Ala  Ala  Leu  Gln
     Glu  Lys  Leu  (Val  Ser Glu)ₘ  Cys  Ala  Thr  Tyr  Lys
     Leu  Cys  His  Pro  Glu  Glu  Leu  Val  Leu  Leu  Gly
     His  Ser  Leu  Gly  Ile  Pro  Trp  Ala  Pro  Leu  Ser
```

```
     Ser  Cys  Pro  Ser  Gln  Ala  Leu  Gln  Leu  Ala  Gly
     Cys  Leu  Ser  Gln  Leu  His  Ser  Gly  Leu  Phe  Leu
     Tyr  Gln  Gly  Leu  Leu  Gln  Ala  Leu  Glu  Gly  Ile
     Ser  Pro  Glu  Leu  Gly  Pro  Thr  Leu  Asp  Thr  Leu
     Gln  Leu  Asp  Val  Ala  Asp  Phe  Ala  Thr  Thr  Ile
     Trp  Gln  Gln  Met  Glu  Glu  Leu  Gly  Met  Ala  Pro
     Ala  Leu  Gln  Pro  Thr  Gln  Gly  Ala  Met  Pro  Ala
     Phe  Ala  Ser  Ala  Phe  Gln  Arg  Arg  Ala  Gly  Gly
     Val  Leu  Val  Ala  Ser  His  Leu  Gln  Ser  Phe  Leu
     Glu  Val  Ser  Tyr  Arg  Val  Leu  Arg  His  Leu  Ala
     Gln  Pro
```

(avec la restriction que m est 0 ou 1 et n est 0 ou 1).

10. Utilisation suivant la revendication 6, dans laquelle la glycoprotéine ayant l'activité de facteur de stimulation de colonie de granulocytes humains comprend un polypeptide et une partie chaîne de sucre, le polypeptide étant représenté par tout ou partie de la séquence d'acides aminés indiquée ci-après :

```
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  (Val Ser  Glu)ₘ Cys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe
Ala  Ser  Ala  Phe  Gln  Arg  Arg  Ala  Gly  Gly  Val
Leu  Val  Ala  Ser  His  Leu  Gln  Ser  Phe  Leu  Glu


Val  Ser  Tyr  Arg  Val  Leu  Arg  His  Leu  Ala  Gln
Pro
```

(avec la restriction que m est 0 ou 1).

**Revendications pour les Etats contractants suivants : AT, SE**

1. Procédé de préparation d'une composition pharmaceutique pour le traitement de la neutropénie par augmentation du nombre de neutrophiles ayant une forme à complète maturité, qui comprend la combinaison d'un facteur de stimulation de colonie de granulocytes humains avec un excipient ou support pharmaceutiquement acceptable et facultativement un stabilisant et/ou un agent antiadsorption.

2. Procédé suivant la revendication 1, dans lequel le facteur de stimulation de colonie de granulocytes humains est présent en une quantité de 0,01 à 500 µg.

3. Procédé suivant les revendications 1 et 2, dans lequel le facteur de stimulation de colonie de granulocytes humains est présent en une quantité de 0,5 à 200 µg.

4. Procédé suivant la revendication 1, dans lequel le facteur de stimulation de colonie de granulocytes humains est obtenu à partir du surnageant de la culture d'une cellule produisant le facteur de stimulation de colonie de granulocytes humains.

5. Procédé suivant la revendication 4, dans lequel le facteur de stimulation de colonie de granulocytes humains a les propriétés physico-chimiques suivantes :

i) poids moléculaire
environ 19 000 ± 1 000 tel que mesuré par électrophorèse dans un gel de polyacrylamide avec dodécylsulfate de sodium;
ii) point isoélectrique :
ayant au moins l'un des trois points isoélectriques, pI = 5,5 ± 0,1, pI = 5,8 ± 0,1 et pI = 6,1 ± 0,1;
iii) absorption dans l'ultraviolet :
ayant un maximum d'absorption à 280 nm et un minimum d'absorption à 250 nm;
iv) séquence d'acides aminés des 21 résidus depuis l'extrémité N terminale :

```
H2N - Thr - Pro - Leu - Gly - Pro - Ala - Ser - Ser -
Leu - Pro - Gln - Ser - Phe - Leu - Leu - Lys - Cys -
Leu - Glu - Gln - Val.
```

6. Procédé suivant la revendication 1, dans lequel le facteur de stimulation de colonie de granulocytes humains est un polypeptide ou une glycoprotéine ayant l'activité de facteur de stimulation de colonie de granulocytes humains qui est produit par un transformant contenant un vecteur recombinant hébergeant un gène codant pour le polypeptide ayant l'activité de facteur de stimulation de colonie de granulocytes humains.

7. Procédé suivant la revendication 6, dans lequel le gène codant pour le polypeptide ayant l'activité de facteur de stimulation de colonie de granulocytes humains a tout ou partie de la séquence de nucléotides représentée à la Fig. 2 annexée.

8. Procédé suivant la revendication 6, dans lequel le gène codant pour le polypeptide ayant l'activité de facteur de stimulation de colonie de granulocytes humains a tout ou partie de la séquence de nucléotides représentée à la Fig. 3 annexée.

9. Procédé suivant la revendication 6, dans lequel le polypeptide ayant l'activité de facteur de stimulation de colonie de granulocytes humains est représenté par tout ou partie de la séquence d'acides aminés indiquée ci-après :

```
(Met) n Thr   Pro   Leu   Gly   Pro   Ala   Ser   Ser   Leu   Pro
        Gln   Ser   Phe   Leu   Leu   Lys   Cys   Leu   Glu   Gln   Val
        Arg   Lys   Ile   Gln   Gly   Asp   Gly   Ala   Ala   Leu   Gln
        Glu   Lys   Leu   (Val  Ser Glu) m Cys   Ala   Thr   Tyr   Lys
        Leu   Cys   His   Pro   Glu   Glu   Leu   Val   Leu   Leu   Gly
```

```
His  Ser  Leu  Gly  Ile  Pro  Trp  Ala  Pro  Leu  Ser
Ser  Cys  Pro  Ser  Gln  Ala  Leu  Gln  Leu  Ala  Gly
Cys  Leu  Ser  Gln  Leu  His  Ser  Gly  Leu  Phe  Leu
Tyr  Gln  Gly  Leu  Leu  Gln  Ala  Leu  Glu  Gly  Ile
Ser  Pro  Glu  Leu  Gly  Pro  Thr  Leu  Asp  Thr  Leu
Gln  Leu  Asp  Val  Ala  Asp  Phe  Ala  Thr  Thr  Ile
Trp  Gln  Gln  Met  Glu  Glu  Leu  Gly  Met  Ala  Pro
Ala  Leu  Gln  Pro  Thr  Gln  Gly  Ala  Met  Pro  Ala
Phe  Ala  Ser  Ala  Phe  Gln  Arg  Arg  Ala  Gly  Gly
Val  Leu  Val  Ala  Ser  His  Leu  Gln  Ser  Phe  Leu
Glu  Val  Ser  Tyr  Arg  Val  Leu  Arg  His  Leu  Ala
Gln  Pro
```

(avec la restriction que m est 0 ou 1 et n est 0 ou 1).

**10.** Procédé suivant la revendication 6, dans lequel la glycoprotéine ayant l'activité de facteur de stimulation de colonie de granulocytes humains comprend un polypeptide et une partie chaîne de sucre, le polypeptide étant représenté par tout ou partie de la séquence d'acides aminés indiquée ci-après :

```
Thr  Pro  Leu  Gly  Pro  Ala  Ser  Ser  Leu  Pro  Gln
Ser  Phe  Leu  Leu  Lys  Cys  Leu  Glu  Gln  Val  Arg
Lys  Ile  Gln  Gly  Asp  Gly  Ala  Ala  Leu  Gln  Glu
Lys  Leu  (Val  Ser  Glu)ₘ Cys  Ala  Thr  Tyr  Lys  Leu
Cys  His  Pro  Glu  Glu  Leu  Val  Leu  Leu  Gly  His
Ser  Leu  Gly  Ile  Pro  Trp  Ala  Pro  Leu  Ser  Ser
Cys  Pro  Ser  Gln  Ala  Leu  Gln  Leu  Ala  Gly  Cys
Leu  Ser  Gln  Leu  His  Ser  Gly  Leu  Phe  Leu  Tyr
Gln  Gly  Leu  Leu  Gln  Ala  Leu  Glu  Gly  Ile  Ser
Pro  Glu  Leu  Gly  Pro  Thr  Leu  Asp  Thr  Leu  Gln
Leu  Asp  Val  Ala  Asp  Phe  Ala  Thr  Thr  Ile  Trp
Gln  Gln  Met  Glu  Glu  Leu  Gly  Met  Ala  Pro  Ala
Leu  Gln  Pro  Thr  Gln  Gly  Ala  Met  Pro  Ala  Phe
Ala  Ser  Ala  Phe  Gln  Arg  Arg  Ala  Gly  Gly  Val
```

```
Leu  Val  Ala  Ser  His  Leu  Gln  Ser  Phe  Leu  Glu
Val  Ser  Tyr  Arg  Val  Leu  Arg  His  Leu  Ala  Gln
Pro
```

(avec la restriction que m est 0 ou 1).

# Fig. 1

## Probe (IWQ)

```
Ile Trp Gln Gln Met Glu Glu Leu Gly Met

5'---- ATI TGG CAA CAA ATG GAA GAA CTI GGI ATG ----3'
           G   G       G   G T
```

## Probe (A)

```
Met Pro Ala Phe Ala

5'---- ATG CCA GCA TTT GC ----3'
           T   T   C
           G   G
           C   C

3'---- TAC GGA CGA AAA CG ----5'        ] Probe(A)
           T   T   G
           G   G
           C   C
```

## Probe (LC)

```
Gln Glu Lys Leu Cys Ala Thr Tyr

5'---- CAG GAG AAG CTG TGT GCC ACC TAC ----3'

3'---- GTC CTC TTC GAC ACA CGG TGG ATG ----5'  Probe(LC)
```

## Fig. 2-1

```
                                                    10                          30
                                    CGGAGCCTGCAGCCCAGCCCCACCCAGACCC


                                    50                          70                          90
ATG GCT GGA CCT GCC ACC CAG AGC CCC ATG AAG CTG ATG GCC CTG CAG CTG CTG CTG TGG
Met Ala Gly Pro Ala Thr Gln Ser Pro Met Lys Leu Met Ala Leu Gln Leu Leu Leu Trp
-30                                                 -20


                                   110                         130                         150
CAC AGT GCA CTC TGG ACA GTG CAG GAA GCC ACC CCC CTG GGC CCT GCC AGC TCC CTG CCC
His Ser Ala Leu Trp Thr Val Gln Glu Ala Thr Pro Leu Gly Pro Ala Ser Ser Leu Pro
-10                                                 -1   1                              10


                                   170                         190                         210
CAG AGC TTC CTG CTC AAG TGC TTA GAG CAA GTG AGG AAG ATC CAG GGC GAT GGC GCA GCG
Gln Ser Phe Leu Leu Lys Cys Leu Glu Gln Val Arg Lys Ile Gln Gly Asp Gly Ala Ala
                                        20                                          30


                                   230                         250                         270
CTC CAG GAG AAG CTG GTG AGT GAG TGT GCC ACC TAC AAG CTG TGC CAC CCC GAG GAG CTG
Leu Gln Glu Lys Leu Val Ser Glu Cys Ala Thr Tyr Lys Leu Cys His Pro Glu Glu Leu
                                        40                                          50


                                   290                         310                         330
GTG CTG CTC GGA CAC TCT CTG GGC ATC CCC TGG GCT CCC CTG AGC AGC TGC CCC AGC CAG
Val Leu Leu Gly His Ser Leu Gly Ile Pro Trp Ala Pro Leu Ser Ser Cys Pro Ser Gln
                                        60                                          70


                                   350                         370                         390
GCC CTG CAG CTG GCA GGC TGC TTG AGC CAA CTC CAT AGC GGC CTT TTC CTC TAC CAG GGG
Ala Leu Gln Leu Ala Gly Cys Leu Ser Gln Leu His Ser Gly Leu Phe Leu Tyr Gln Gly
                                        80                                          90
```

# Fig. 2-2

```
              410                          430                          450
CTC CTG CAG GCC CTG GAA GGG ATC TCC CCC GAG TTG GGT CCC ACC TTG GAC ACA CTG CAG
Leu Leu Gln Ala Leu Glu Gly Ile Ser Pro Glu Leu Gly Pro Thr Leu Asp Thr Leu Gln
                                         100                                  110


              470                          490                          510
CTG GAC GTC GCC GAC TTT GCC ACC ACC ATC TGG CAG CAG ATG GAA GAA CTG GGA ATG GCC
Leu Asp Val Ala Asp Phe Ala Thr Thr Ile Trp Gln Gln Met Glu Glu Leu Gly Met Ala
                                         120                                  130


              530                          550                          570
CCT GCC CTG CAG CCC ACC CAG GGT GCC ATG CCG GCC TTC GCC TCT GCT TTC CAG CGC CGG
Pro Ala Leu Gln Pro Thr Gln Gly Ala Met Pro Ala Phe Ala Ser Ala Phe Gln Arg Arg
                                         140                                  150


              590                          610                          630
GCA GGA GGG GTC CTG GTT GCC TCC CAT CTG CAG AGC TTC CTG GAG GTG TCG TAC CGC GTT
Ala Gly Gly Val Leu Val Ala Ser His Leu Gln Ser Phe Leu Glu Val Ser Tyr Arg Val
                                         160                                  170


              650                          670                          690
CTA CGC CAC CTT GCC CAG CCC TGA GCCAAGCCCTCCCCATCCCATGTATTTATCTCTATTTAATATTTATG
Leu Arg His Leu Ala Gln Pro End


       710             730             750             770
TCTATTTAAGCCTCATATTTAAAGACAGGGAAGAGCAGAACGGAGCCCCAGGCCTCTGTGTCCTTCCCTGCATTTCTG


       790             810             830             850
AGTTTCATTCTCCTGCCTGTAGCAGTGAGAAAAAGCTCCTGTCCTCCCATCCCCTGGACTGGGAGGTAGATAGGTAAAT


       870             890             910             930
ACCAAGTATTTATTACTATGACTGCTCCCCAGCCCTGGCTCTGCAATGGGCACTGGGATGAGCCGCTGTGAGCCCCTGG
```

EP 0 217 404 B2

# Fig. 2-3

TCCTGAGGGTCCCCACCTGGGACCCTTGAGAGTATCAGGTCTCCCACGTGGGAGACAAGAAATCCCTGTTTAATATTTA

AACAGCAGTGTTCCCCATCTGGGTCCTTGCACCCCTCACTCTGGCCTCAGCCGACTGCACAGCGGCCCCTGCATCCCCTT

GGCTGTGAGGCCCCTGGACAAGCAGAGGTGGCCAGAGCTGGGAGGCATGGCCCTGGGGTCCCACGAATTTGCTGGGGAA

TCTCGTTTTTCTTCTTAAGACTTTTGGGACATGGTTTGACTCCCGAACATCACCGACGCGTCTCCTGTTTTTCTGGGTG

GCCTCGGGACACCTGCCCTGCCCCCACGAGGGTCAGGACTGTGACTCTTTTTAGGGCCAGGCAGGTGCCTGGACATTTG

CCTTGCTGGACGGGGACTGGGGATGTGGGAGGGAGCAGACAGGAGGAATCATGTCAGGCCTGTGTGTGAAAGGAAGCTC

CACTGTCACCCTCCACCTCTTCACCCCCCACTCACCAGTGTCCCCTCCACTGTCACATTGTAACTGAACTTCAGGATA

ATAAAGTGTTTGCCTCCAAAAAAAAAAAAAAAAAAAAAAAA

# Fig. 3-1

```
                                                    10                            30
                                   GGAGCCTGCAGCCCAGCCCCACCCAGACCC
```

```
                            50                                  70                          90
ATG GCT GGA CCT GCC ACC CAG AGC CCC ATG AAG CTG ATG GCC CTG CAG CTG CTG CTG TGG
Met Ala Gly Pro Ala Thr Gln Ser Pro Met Lys Leu Met Ala Leu Gln Leu Leu Leu Trp
-30                                                 -20
```

```
                                   110                         130                        150
CAC AGT GCA CTC TGG ACA GTG CAG GAA GCC ACC CCC CTG GGC CCT GCC AGC TCC CTG CCC
His Ser Ala Leu Trp Thr Val Gln Glu Ala Thr Pro Leu Gly Pro Ala Ser Ser Leu Pro
-10                                                 -1    1                               10
```

```
                                   170                         190                        210
CAG AGC TTC CTG CTC AAG TGC TTA GAG CAA GTG AGG AAG ATC CAG GGC GAT GGC GCA GCG
Gln Ser Phe Leu Leu Lys Cys Leu Glu Gln Val Arg Lys Ile Gln Gly Asp Gly Ala Ala
                                             20                                         30
```

```
                            230                                 250                        270
CTC CAG GAG AAG CTG TGT GCC ACC TAC AAG CTG TGC CAC CCC GAG GAG CTG GTG CTG CTC
Leu Gln Glu Lys Leu Cys Ala Thr Tyr Lys Leu Cys His Pro Glu Glu Leu Val Leu Leu
                                       40                                              50
```

```
                            290                                 310                        330
GGA CAC TCT CTG GGC ATC CCC TGG GCT CCC CTG AGC AGC TGC CCC AGC CAG GCC CTG CAG
Gly His Ser Leu Gly Ile Pro Trp Ala Pro Leu Ser Ser Cys Pro Ser Gln Ala Leu Gln
                                       60                                              70
```

```
                            350                                 370                        390
CTG GCA GGC TGC TTG AGC CAA CTC CAT AGC GGC CTT TTC CTC TAC CAG GGG CTC CTG CAG
Leu Ala Gly Cys Leu Ser Gln Leu His Ser Gly Leu Phe Leu Tyr Gln Gly Leu Leu Gln
                                       80                                              90
```

EP 0 217 404 B2

# Fig. 3-2

```
                      410                                    430                                   450
GCC CTG GAA GGG ATC TCC CCC GAG TTG GGT CCC ACC TTG GAC ACA CTG CAG CTG GAC GTC
Ala Leu Glu Gly Ile Ser Pro Glu Leu Gly Pro Thr Leu Asp Thr Leu Gln Leu Asp Val
                                          100                                               110


                      470                                    490                                   510
GCC GAC TTT GCC ACC ACC ATC TGG CAG CAG ATG GAA GAA CTG GGA ATG GCC CCT GCC CTG
Ala Asp Phe Ala Thr Thr Ile Trp Gln Gln Met Glu Glu Leu Gly Met Ala Pro Ala Leu
                                          120                                               130


                      530                                    550                                   570
CAG CCC ACC CAG GGT GCC ATG CCG GCC TTC GCC TCT GCT TTC CAG CGC CGG GCA GGA GGG
Gln Pro Thr Gln Gly Ala Met Pro Ala Phe Ala Ser Ala Phe Gln Arg Arg Ala Gly Gly
                                          140                                               150


                      540                                    610                                   630
GTC CTA GTT GCC TCC CAT CTG CAG AGC TTC CTG GAG GTG TCG TAC CGC GTT CTA CGC CAC
Val Leu Val Ala Ser His Leu Gln Ser Phe Leu Glu Val Ser Tyr Arg Val Leu Arg His
                                          160                                               170


                   650                     670                     690
CTT GCC CAG CCC TGA GCCAAGCCCTCCCCATCCCATGTATTTATCTCTATTTAATATTTATGTCTATTTAAGCC
Leu Ala Gln Pro End


    710                     730                     750                     770
TCATATTTAAAGACAGGGAAGAGCAGAACGGAGCCCCAGGCCTCTGTGTCCTTCCCTGCATTTCTGAGTTTCATTCTCC


    790                     810                     830                     850
TGCCTGTAGCAGTGAGAAAAAGCTCCTGTCCTCCCATCCCCTGGACTGGGAGGTAGATAGGTAAATACCAAGTATTTAT
```

## Fig. 3-3

```
      870                    890                    910                    930
TACTATGACTGCTCCCCAGCCCTGGCTCTGCAATGGGCACTGGGATGAGCCGCTGTGAGCCCCTGGTCCTGAGGGTCCC

      950                    970                    990                   1010
CACCTGGGACCCTTGAGAGTATCAGGTCTCCCACGTGGGAGACAAGAAATCCCTGTTTAATATTTAAACAGCAGTGTTC

     1030                   1050                   1070                   1090
CCCATCTGGGTCCTTGCACCCCTCACTCTGGCCTCAGCCGACTGCACAGCGGCCCCTGCATCCCCTTGGCTGTGAGGCC

     1110                   1130                   1150                   1170
CCTGGACAAGCAGAGGTGGCCAGAGCTGGGAGGCATGGCCCTGGGGTCCCACGAATTTGCTGGGGAATCTCGTTTTTCT

     1190                   1210                   1230                   1250
TCTTAAGACTTTTGGGACATGGTTTGACTCCCGAACATCACCGACGCGTCTCCTGTTTTTCTGGGTGGCCTCGGGACA

     1270                   1290                   1310                   1330
CCTGCCCTGCCCCCACGAGGGTCAGGACTGTGACTCTTTTTAGGGCCAGGCAGGTGCCTGGACATTTGCCTTGCTGGAC

     1350                   1370                   1390                   1410
GGGGACTGGGGATGTGGGAGGGAGCAGACAGGAGGAATCATGTCAGGCCTGTGTGTGAAAGGAAGCTCCACTGTCACCC

     1430                   1450                   1470                   1490
TCCACCTCTTCACCCCCCACTCACCAGTGTCCCCTCCACTGTCACATTGTAACTGAACTTCAGGATAATAAAGTGCTTG

     1510
CCTCCAAAAAAAAAAAAAAAAAAAAAAAAAA
```

Fig. 4

Fig. 5

Fig. 6

## Fig. 7